# EUROPÄISCHE PATENTSCHRIFT

(11) **EP 1 252 163 B1**
(45) Veröffentlichungstag und Bekanntmachung des Hinweises auf die Patenterteilung: **24.09.2003**
(21) Anmeldenummer: 01902370.4
(22) Anmeldetag: 31.01.2001
(51) Int. Cl.: C07D 487/04, A61K 31/395, C07D 513/04, C07D 471/04, C07D 498/04

(54) **4-PYRIDYL- UND 2,4-PYRIMIDINYL-SUBSTITUIERTE PYRROLDERIVATE UND IHRE ANWENDUNG IN DER PHARMAZIE**
4-PYRIDYL-AND 2,4-PYRIMIDINYL-SUBSTITUTED PYRROLE DERIVATIVES AND THEIR USE IN PHARMACY
DERIVES DE PYRROLE 4-PYRIDYL- ET 2,4-PYRIMIDINYL-SUBSTITUES ET LEUR UTILISATION EN PHARMACIE

(30) Priorität: 01.02.2000 DE 10004157
(43) Veröffentlichungstag der Anmeldung: 30.10.2002
(73) Patentinhaber: MERCKLE GMBH, D-89079 Ulm (DE)
(72) Erfinder: STRIEGEL, Hans-Günter, 89134 Blaustein (DE); LAUFER, Stefan, 89143 Blaubeuren (DE); TOLLMANN, Karola, 65611 Brechen (DE); TRIES, Susanne, 89584 Ehingen (DE)
(74) Vertreter: Riedl, Peter, Dr.
(86) Internationale Anmeldenummer: EP0101011
(87) Internationale Veröffentlichungsnummer: WO01057042

(56) Entgegenhaltungen:
- WO-A-95/32972
- US-A- 5 583 148

## Beschreibung

Die vorliegende Erfindung betrifft 4-Pyridyl- und 2,4-Pyrimidinyl-substituierte Pyrrolderivate mit immunmodulierender und die Cytokinfreisetzung hemmender Wirkung, pharmazeutische Mittel, welche diese Verbindungen enthalten, und ihre Anwendung in der Pharmazie.

Pharmakologisch wirksame Pyrrolizinverbindungen, welche die 5-Lipoxygenase (5-LO) und die Cyclooxygenase-1 und -2 (Cox-1 und

Cox-2) inhibieren, sind bereits bekannt.
Beispielsweise werden antiphlogistisch wirksame Pyrrolizin-Verbindungen beschrieben in Arch. Pharm. 319, 231-234 (1986), 318, 661-663 (1985), 318, 663-664 (1985), 319, 500-505 (1986), 319, 749-755 (1986), 327, 509-514 (1994), 330, 307-312 (1997) sowie in J. Med. Chem. 1987, 30, 820-823 und 1994, 37, 1894-1897. Die erfolgversprechendste Verbindung dieses Typs ist die 6-(4-Chlorphenyl)-7-phenyl-2,3-dihydropyrrolo[1,2-a]pyrrol-Verbindung ML 3000, s. Drugs of the Future, 1995, 20, (10).: 1007-1009. Sie unterdrückt die Freisetzung von Leukotrienen, Thromboxanen und Prostaglandinen. Die inhibitorische Wirkung auf die Bildung der Leukotrienen und der Prostaglandine ist bei dieser Struktur ausgewogen, schädliche Effekte einer reinen Hemmwirkung auf Cyclooxygenase -1 und -2 (Cox-1 bzw. Cox-2) mit vermehrter Bildung von Leukotrienen werden hier nicht beobachtet. Bei allen diesen Verbindungen ist die 1-Stellung des Pyrrolizingerüstes unsubstituiert.

Die WO 95/32970, Wo 95/32971 und WO 95/32972 betreffen Verbindungen der Formel, 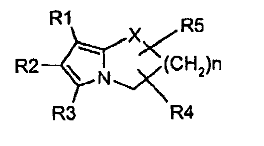 wobei
einer oder zwei der Reste R¹, R² und R³ einen mono- oder bicyclischen, aromatischen, heterocyclischen Rest, der mindestens ein Sauerstoff-, Stickstoff- und/oder Schwefelatom aufweist, bedeuten können. Diese Verbindungen besitzen antiinflammatorische Wirkung.

Weitere anellierte Pyrrolverbindungen und strukturell ähnliche Verbindungen sind beschrieben in US 5,260,451,US 4,546,100 und US 4, 584, 297; US 4, 684, 658; US 5,631,122; US 3,920,672; US 4,536,512; US 5,552,422; US 4,539,400; US 4,719,218; EP 608 133 A, DE 198 45 446 A, PCT/EP 99/09057 und DE 100 01 166. Es ist nicht offenbart, dass diese Verbindungen immunmodulierende oder die Cytokinfreisetzung hemmende Wirkung aufweisen.

Die US 5,583,148 beschreibt bis-Acyloxymethylpyridyl-Verbindungen und entsprechende Chinolin- und Acridinderivate auf Basis eines Pyrrolizingerüstes. Diese Verbindungen sind als Fungizide, Bakterizide und zur Hemmung des Tumorwachstums brauchbar.

überraschenderweise wurde nun gefunden, dass bestimmte anellierte Pyrrolverbindungen, die am Pyrrolring einen 4-Pyridyl-Substituenten aufweisen, immunmodulierende und/oder die Cytokinfreisetzung hemmende Wirkung aufweisen.

Gegenstand der vorliegenden Erfindung sind daher die 4-Pyridyl- und 2,4-Pyrimidinyl-substituierte Pyrrolderivate und ihre Anwendung in der Pharmazie der Formel I 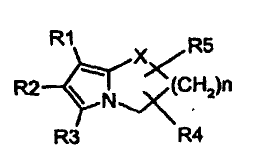 worin
einer der Reste R¹, R² und R³ für eine Gruppe der Formel 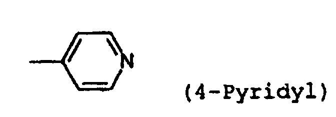 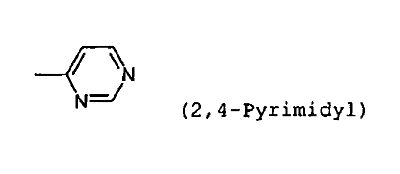 oder 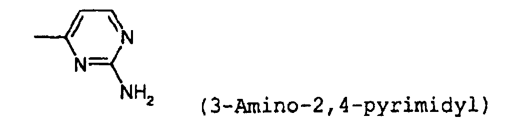 steht, die gegebenenfalls durch ein oder zwei C₁-C₄-Alkylgruppen oder Halogenatome substituiert ist,
der zweite der Reste R¹, R² und R³ für Phenyl oder Thienyl steht, das gegebenenfalls durch ein oder zwei Halogenatome substituiert ist, und
der dritte der Reste R¹, R² und R³ für H, CO₂H, CO₂C₁-C₆-Alkyl, CH₂OH oder C₁-C₆-Alkyl steht,
R⁴ und R⁵ unabhängig voneinander für H oder C₁-C₆-Alkyl stehen,
X für CH₂, S oder O steht und
n für 1 oder 2 steht,
und die optischen Isomere physiologisch verträglichen Salze und physiologisch leicht hydrolisierbaren Ester davon.

Die physiologisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Citronensäure, Maleinsäure, Fumarsäure, Äpfelsäure, Mandelsäure, Ascorbinsäure, Gluconsäure und dergleichen.

Zu Basenadditionssalzen zählen Salze der Verbindungen der Formel I mit anorganischen Basen, wie Natrium- oder Kaliumhydroxid, oder mit organischen Basen, wie Mono-, Di- oder Triethanolamin.

Physiologisch leicht hydrolysierbare Ester der Verbindungen der Formel I sind beispielsweise Alkyl-, Pivaloyloxymethyl-Acetoxymethyl, Phthalidyl, Indanyl- und Methoxymethylester.

Soweit die erfindungsgemäßen Verbindungen Asymmetriezentren aufweisen, sind Racemate sowie optische Isomere (Enantiomere, Diastereomere) umfasst.

Der Ausdruck "C₁-C₆-Alkyl" umfasst geradkettige oder verzweigte Alkylgruppen, wie Methyl, Ethyl, n-Propyl, i-Propyl, n-, i-oder t- Butyl, sec-Butyl, n-Pentyl und n-Hexyl.

Der Ausdruck "Halogen" umfasst ein Fluor-, Chlor- Brom- oder Iodatom und insbesondere ein Fluor- oder Chloratom.

Wenn der zweite der Reste R¹, R² und R³ für Phenyl steht, ist dieses vorzugsweise durch ein Halogenatom, insbesondere ein Fluoratom, substituiert. Bevorzugt befindet sich das Halogenatom in 4-Postion.

Wenn der zweite der Reste R¹, R² und R³ für Thienyl steht, ist dieses vorzugsweise in 2-Stellung gebunden. Wenn die 2-Thienylgruppe substituiert ist, ist sie vorzugsweise mit einem Halogenatom und insbesondere in 5-Position substituiert.

Die Herstellung der erfindungsgemäßen Verbindungen erfolgt in Abhängigkeit von der Stellung der aromatischen und in Abhängigkeit von der Art der heteroaromatischen Reste R¹, R² und R³ nach verschiedenen Verfahren.
Die [α]-heterocyclisch anellierten Pyrrolverbindungen, bei denen für X = S, oder O steht, werden analog zu den in WO 95/32970, WO 95/32971 und WO 95/32972 beschriebenen Verfahren hergestellt.

Die Beispiele 23 und 24 beschreiben, wie 5H-Furan-2-on-Vorstufen mit den essigsauren Salzen von Aminoalkoholen zu 1-Hydroxy-Alkyl-2-pyrrolonen kondensiert werden, welche mit geeigneten Kondensationsmitteln (hier P₂S₅ bzw. Methansulfonsäurechlorid) die [α]-heterocyclisch anellierten Pyrrolverbindungen ergeben (Schema 1).

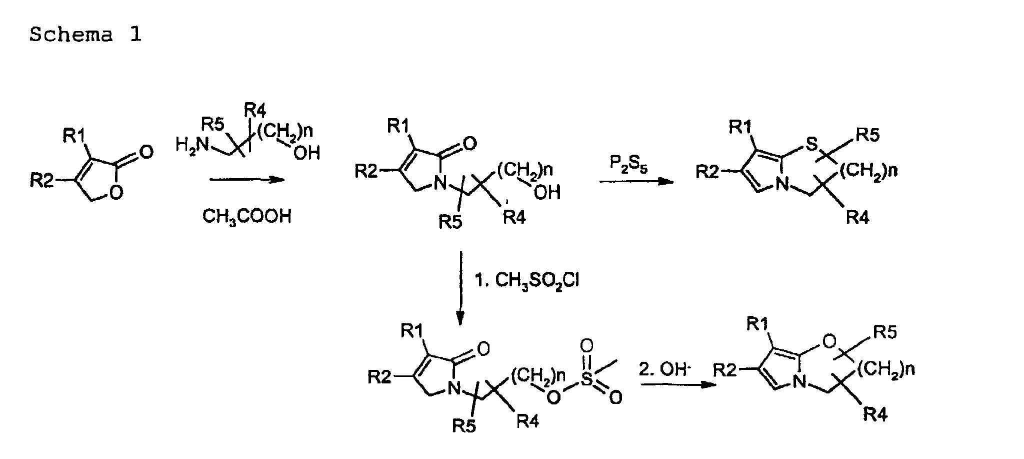

Der Aufbau der Pyrrolizine, Indolizine und deren 1-Thia-Analogen, mit bevorzugter Stellung von Pyridin und Pyrimidin-Rest in Position 5 bzw. 6 (R³) und 6 bzw. 7 (R²), erfolgt auf dem Weg einer 1,3-dipolaren Cycloaddition aus entsprechenden Münchnon- bzw. Sydnon-Vorläuferverbindungen und geeigneten Dienophilen oder Dipolarophilen (Schema 2).

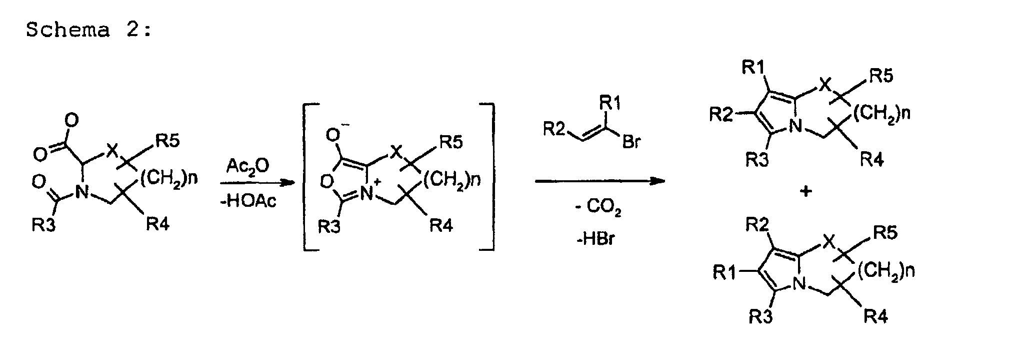

Dabei kann ein Pyridin oder Pyrimidin-Rest einerseits über die Münchnon-/Sydnon-Komponente, andererseits über die Dipolarophilkomponente eingeführt werden. Als Dipolarophil finden Dehydrozimtsäureester, 3-substituierte Acetylencarbonsäureester, 2-halogensubstituierte Zimtsäuren oder 2-Halogenacrylate und Nitrostyrole Verwendung.

In der Reihe der Pyrrolizine werden zur Sydnonbildung N-Acylderivate des Prolin (Pyrrolidin-2-carbonsäure, vgl. Beispiele 1-9, 19, 20, 22), in der Reihe der Thiazolo[2,1-b]pyrrole (vgl. Beispiele 10 - 14) die N-Acylderivate der [1,3]-thiazolidin-2-carbonsäure) und in der Reihe der Indolizine N-Acylderivate der homologen Piperidin-2-carbonsäure (vgl. Beispiel 15-18) eingesetzt.

Beispielsweise führt die Cycloaddition des 2-Brom-3-(4-pyridyl)-propensäureethyiesters an das in situ erzeugte Münchnon des N-(4-Fluorbenzoyl)-prolin zum Ester aus Beispiel 1, des 2-Brom-3-(4-fluorphenyl)-propensäureethylesters an das Münchnon des N-(Isonicotinoyl)-prolin zum Ethylester der Pyrrolizincarbonsäure des Beispiels 19 und die Cycloaddition eines 2-Brom-3-(4-fluorphenyl)-propensäureethylesters an die intramolekular cyclisierte 3-(4-Fluorbenzoyl)-[1,3]-thiazolidin-2-carbonsäure führt zur Pyrrolizin-Verbindung aus Beispiel 10.

Durch den Einsatz von 1-Nitrostyrolen und N-Aroyl-Prolin gelangt man direkt zu 7-(bzw. 1-)unsubstituierten 5,6- (bzw. 2,3-)Diarylpyrrolizin-Verbindungen.
Die Umsetzung des 1-Fluor-4-(2-nitrovinyl)-benzol mit 1-Pyridin-4-carbonyl-pyrrolidin-2-carbonsäure (N-Isonicotinoylprolin) führt zur Verbindung aus Beispiel 22.

Pyridin- und Pyrimidinsubstituenten lassen sich auch nachträglich in die aktivierten Pyrrol-Positionen der Pyrrolizine und Indolizine und Ihrer Thia- und Oxa-Analoga einführen (Schema 3 und 4). Die Umsetzung des 6-(4-Fluorphenyl)-7-methyl-2,3-dihydro-1H-pyrrolizins mit dem aus Chlorameisensäureethylester und Pyridin erhaltenen reaktiven 1-Ethoxycarbonyl-pyridiniumchlorid ergibt die Verbindung des Beispiels 21 (R¹ = CH₃, R² = 4-Fluorphenyl).

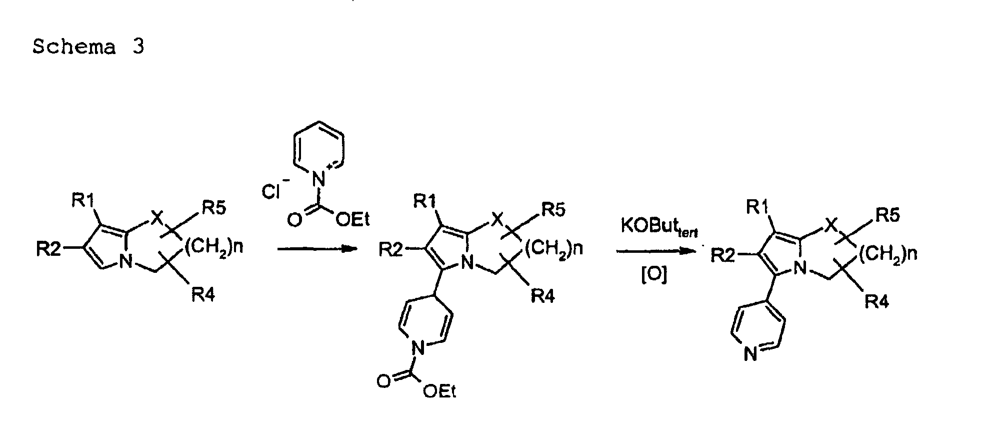

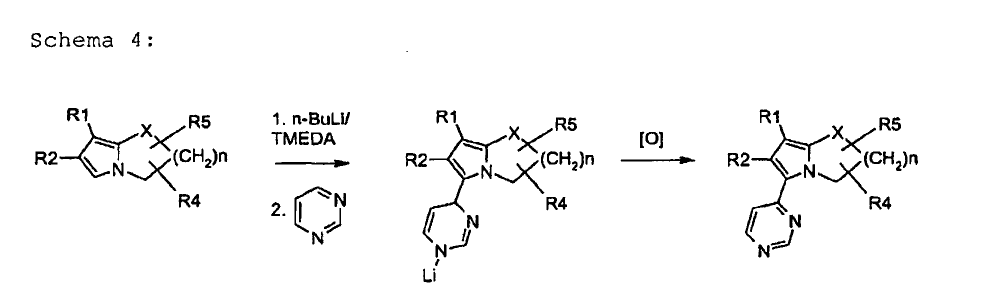

Insbesondere der 3-Amino-2,4-pyrimidinsubstituent kann ausgehend von den Acyl-derivaten der Monoaryl-substituierten Verbindungen über Kondensation mit Dimethylformamiddimethylacetal und Guanidin aufgebaut werden (Schema 5). Der 3-Amino-2,4-pyrimidinsubstituent lässt sich nach dieser Methode in jede reaktive, unsubstituierte Position des Pyrrolringes des Pyrrolizidin und Indolizidinsystems einführen.

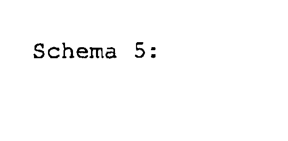

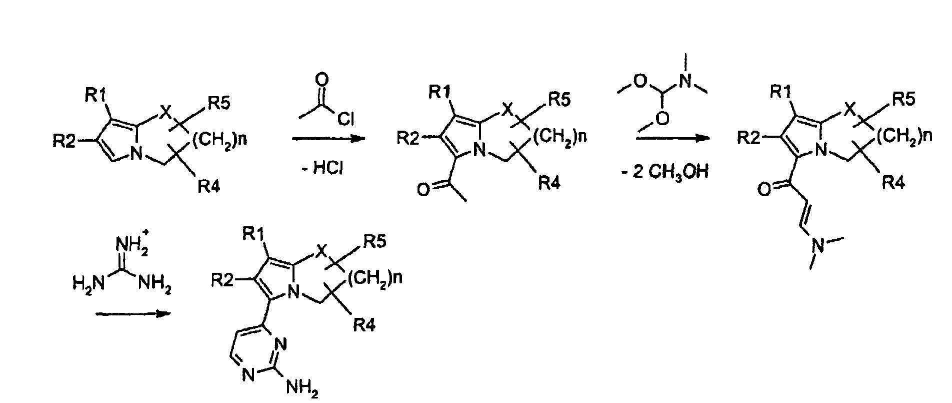

Die erfindungsgemäßen Verbindungen zeigen in vitro und in vivo immunmodulierende und die Cytokinfreisetzung hemmende Wirkung. Sie eignen sich somit zur Herstellung eines pharmazeutischen Mittels zur Behandlung von Erkrankungen, die im Zusammenhang mit einer Störung des Immunsystems stehen. Beispielsweise sind sie brauchbar zur Behandlung von Autoimmunerkrankungen, Krebs, Multipler Sklerose, Arthritis, Inflammatory Bowel Disease, Septischem Schock, Adult Respiratory Distress Syndrome, und bei Transplantationen.

Die erfindungsgemäßen Verbindungen können entweder als einzelne therapeutische Wirkstoffe oder als Mischungen mit anderen therapeutischen Wirkstoffen verabreicht werden. Sie können als solche verabreicht werden, im Allgemeinen werden sie jedoch in Form pharmazeutischer Mittel verabreicht, d.h. als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calziumphosphat, Sorbit oder Glycin), Gleitmittel (z.B. Magnesiumstearat, Talcum, Polyethylenglycol oder Siliziumdioxid), desintegrierende Mittel (z.B. Stärke) oder Netzmittel (z.B. Natriumlaurylsulfat). Orale flüssige Präparate können in Form wässriger oder öliger Suspensionen, Lösungen, Emulsionen, Sirupen, Elixieren oder Sprays usw. oder als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden. Das Wirkungsspektrum der Verbindungen wurde anhand folgender Testsysteme untersucht.

### In vitro-Testverfahren mit humanem Vollblut

Humanes Kalium-EDTA-Vollblut (à 400 µl) wird mit Testsubstanz 15 min. bei 37°C in einem CO₂-Inkubator (5% CO₂; 95% feuchtigkeitsgesättigte Luft) vorinkubiert. Danach werden die Proben 4 Stunden mit 1 µg/ml LPS (*E.coli* 026:B6) bei 37°C im CO₂-Inkubator (5% CO₂; 95% feuchtigkeitsgesättigte Luft) stimuliert. Die Reaktion wird durch Stellen der Proben auf Eis, Zugabe von DPBS-Puffer und anschließende Zentrifugation (1000*g; 15 min) beendet. Der Plasmaüberstand wird zur Quantifizierung von IL-1β und TNFα mittels ELISA verwendet.

### In vitro-Testverfahren mit PBMCs

Die mononukleären Zellen (PBMCs) werden aus 1:3 verdünntem humanen Kalium-EDTA-Vollblut mittels Dichtegradientenzentrifugation (Histopaque®-1.077) isoliert. Nach 2 Waschschritten mit DPBS-Puffer werden die mononuklearen Zellen in Makrophagen-SFM Medium resuspendiert und auf eine Zellzahl von 1*10⁶ Zellen/ml eingestellt.
Die PBMCs-Suspension (à 390 µl) wird mit Testsubstanz 15 min bei 37°C in einem CO₂-Inkubator (5% CO₂; 95% feuchtigkeitsgesättigte Luft) vorinkubiert. Anschließend werden die Proben 4 Stunden mit 1 µg/ml LPS (*E.coli* 026:B6) bei 37°C im CO₂-Inkubator (5% CO₂, 95% feuchtigkeitsgesättigte Luft) stimuliert. Die Reaktion wird durch Stellen der Proben auf Eis, Zugabe von DPBS-Puffer und Zentrifugation (15880*g; 12 min) beendet. Der Überstand wird zur Quantifizierung von IL-1β und TNFα mittels ELISA verwendet.

### In Vitro Testsystem zur Bestimmung der Hemmung der 5-Lipoxygenase

Als Quelle für die 5-Lipoxygenase dienen menschliche Granulozyten. Durch Stimulation mit Calcium-Ionophor A 23187 wird LTB4 (Leukotrien B4) aus endogener Arachidonsäure gebildet. Die Isolierung der Granulozyten und die Durchführung der Enzymreaktion erfolgt nach bekannten Verfahren (siehe Arch. Pharm. Pharm. Med. Chem. 330, 307 - 312 (1997)).

Das mit Heparin vor Gerinnung geschützte Blut wird über einem diskontinuierlichen Percoll®-Gradienten zentrifugiert und die Granulozytenschicht abpipettiert. Nach Lyse der Erythrozyten werden die Granulozyten mehrmals gewaschen und anschließend auf eine bestimmte Zellzahl eingestellt. Die Enzymreaktion wird dann in An- bzw. Abwesenheit der Testsubstanz nach Zugabe von Ca2+ mit Calcium-Ionophor A 23187 gestartet. Die Synthese der Leukotriene wird nach 1,5 Minuten gestoppt. Die Proben werden abzentrifugiert, der Überstand verdünnt. Die quantitative Bestimmung von LTB4 erfolgt mittels ELISA.

### In vitro Testsystem zur Bestimmung der Hemmung der Cyclooxygenase-1

Bei diesem Testsystem wird die von menschlichen Thrombozyten nach Zusatz von Calcium-Ionophor gebildete Prostaglandin E2-Menge mittels ELISA bestimmt. Dabei werden die Thrombozyten nach Zentrifugation über einen diskontinuierlichen Percoll®-Gradienten gewonnen. Die Enzymreaktion und die Bestimmung der gebildeten Metaboliten erfolgt prinzipiell wie bei der Bestimmung der 5-Lipoxygenase-Hemmung. Unterschiede bestehen hinsichtlich der Inkubationszeit. Weiterhin ist die Zugabe eines Thromboxansynthase-Hemmstoffes notwendig (siehe Arch. Pharm. Pharm. Med. Chem. 330, 307 - 312 (1997)).

### In vitro Testsystem zur Bestimmung der Hemmung der Cyclooxygenase-2

COX2 (aus Placenta des Schafs) wird mit Testsubstanz 10 min bei 4°C vorinkubiert, dann mit Arachidonsäure (5 µM) bei 25°C 10 min stimuliert. Als Referenz dient Diclofenac (IC50(COX2) = 3,0 10⁻⁶ M). Die Bestimmung erfolgt in 3 Verdünnungen (10⁻⁷, 10⁻⁶, 10⁻⁵ M). Die PGE2-Konzentrationen werden mittels ELISA quantifiziert (siehe Mitchell J.A. et al., Proc. Nat. Acad. Sci 90: 11693-11697 (1993)).

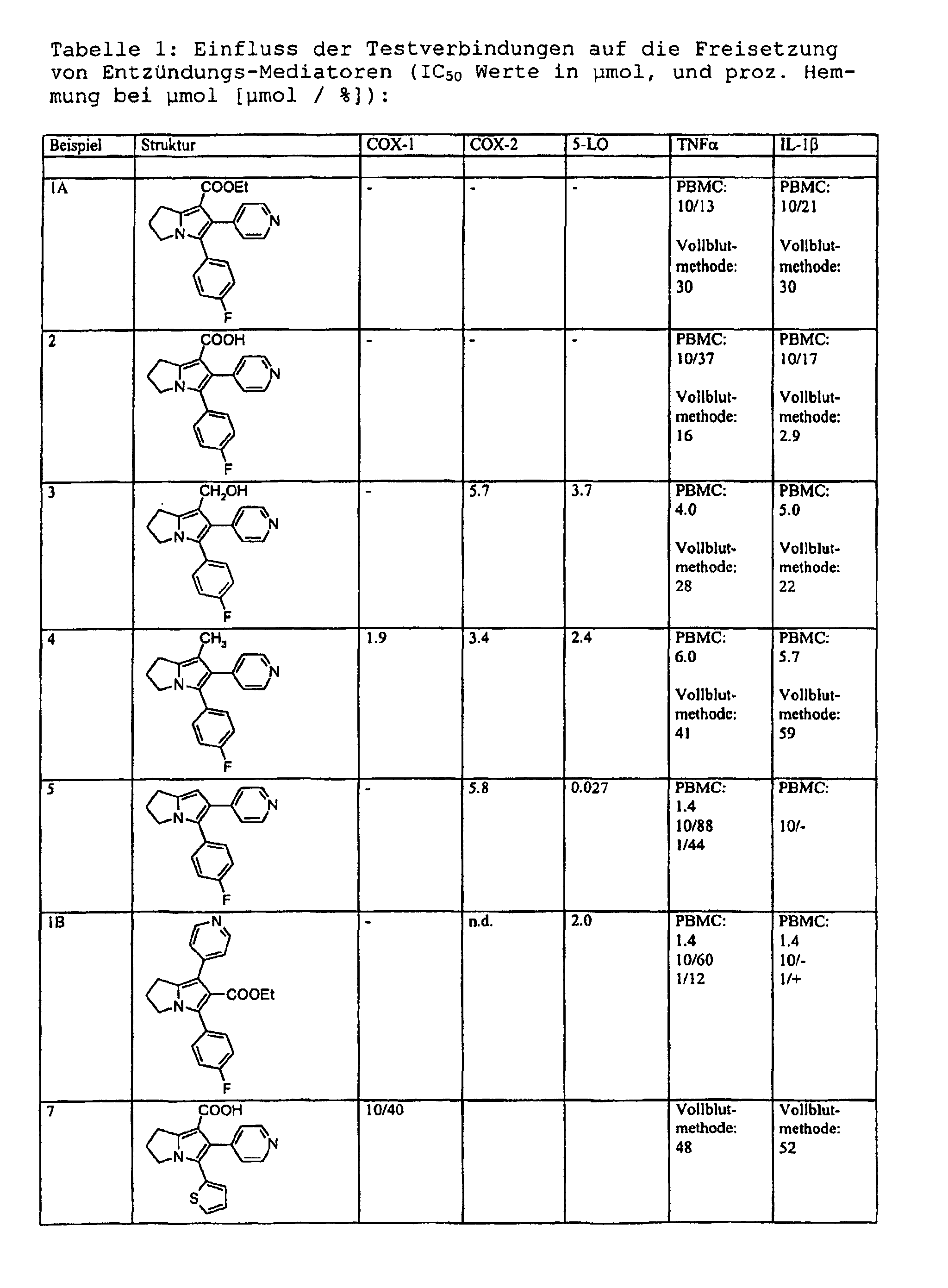

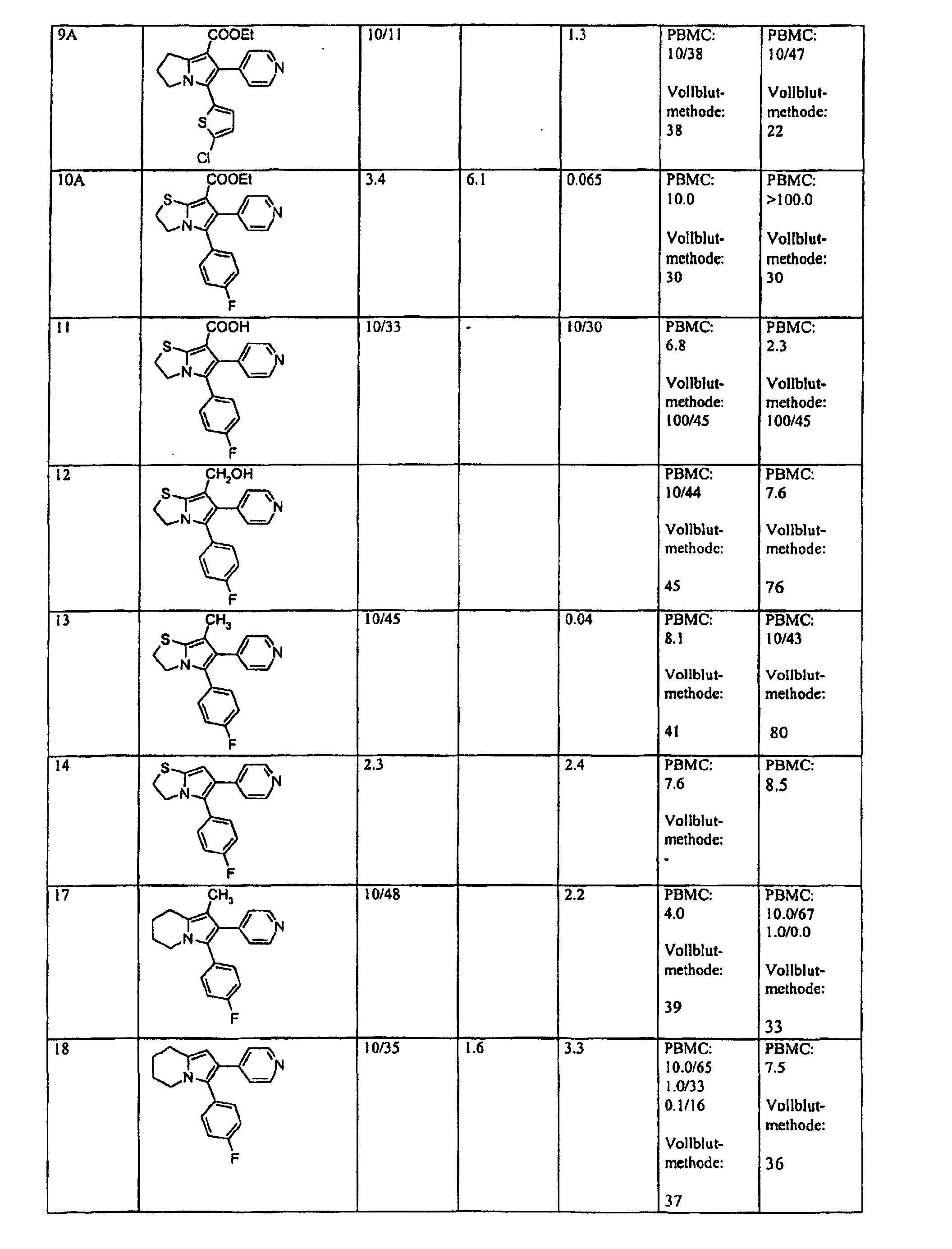

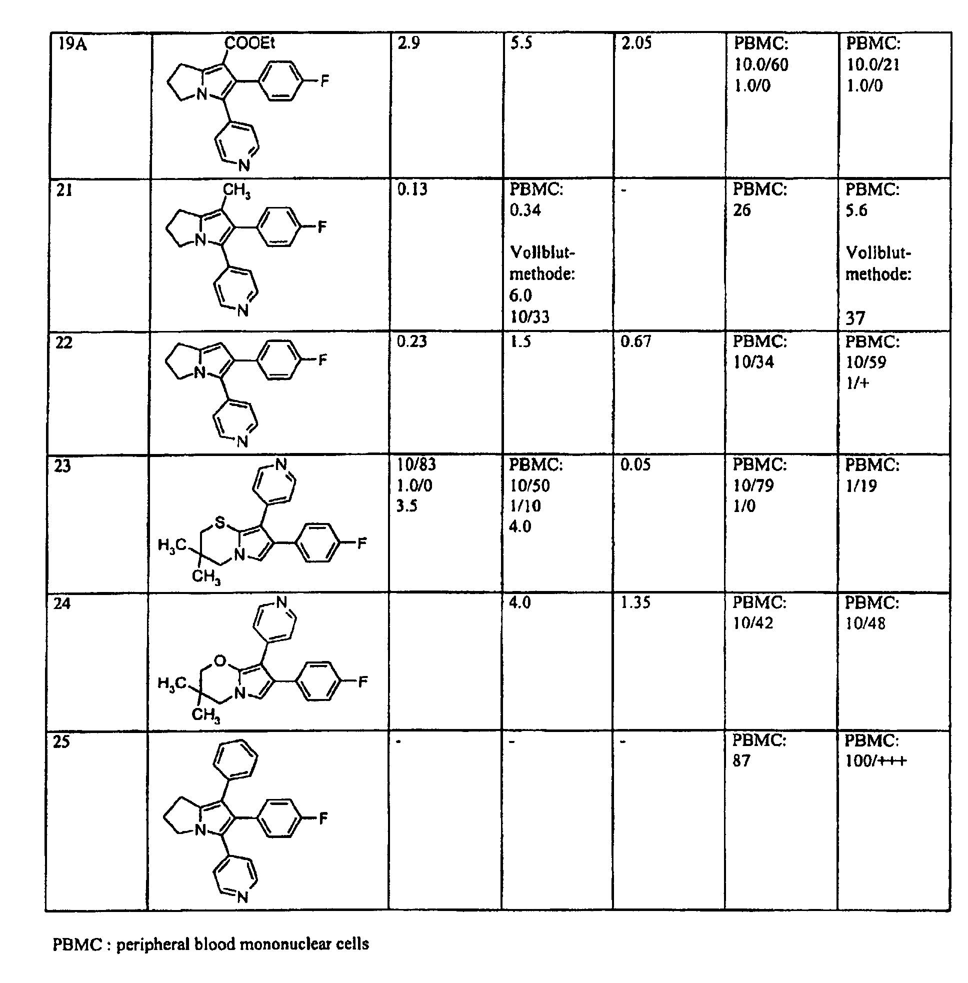

### Beispiel 1A: 3-(4-Fluorphenyl)-2-(4-pyridyl)-6,7-dihydro-5Hpyrrolizin-1-carbonsäureethylester

### a) N-(4-Fluorbenzoyl)-prolin

L-Prolin (15.0 g, 130 mMol) wird in NaOH (5 %, 150 mL, 190 mMol) gelöst und die Lösung im Eisbad abgekühlt (0-5°C). Unter starkem Rühren wird 4-Fluorbenzoylchlorid (19.0 g, 120 mMol) zugetropft, die Kühlung entfernt und 1 h weitergerührt. Nach Ansäuern der Reaktionsmischung mit HCl (10%ig, 45 mL) setzt sich das Amid in großen Klumpen ab, die zerkleinert, mit Wasser aufgeschlämmt, abgesaugt und mit Wasser (50 mL) gewaschen werden. Die Substanz wird im Vakuum über P₂O₅ getrocknet: 24.41 g.
Schmp.: 174.0°C, Ausbeute: 79 %; C₁₂H₁₂FNO₃; MW = 237.23.
IR (KBr): 1/λ (cm⁻¹) = 1735, 1605, 1585, 1514, 1440, 1230, 1180, 1161, 856, 762 513; -
¹H-NMR (CDCl₃:) δ[ppm] = 7.64 - 7.57 (m, 2H, ) ; 7.16 - 7.07 (m, 2H); 4.78 - 4.71 (m, CH); 3.63 - 3.57(CH₂); 2.36 - 1.85 (m; 2 CH₂) .

### b) Brom-carbethoxymethylen-triphenyl-phosphoran

Carboethoxymethyl-triphenylphosphoniumbromid (43 g, 100 mMol) wird in Wasser (100 mL) gelöst und im Eisbad gekühlt. Bei 0-5°C wird NaOH (10%, 40 mL, 100 mMol) zugetropft, bis ein pH von 9 erreicht ist. Die abgeschiedene harzige Masse wird abgetrennt, mit Ether digeriert und die gebildeten Kristalle abgesaugt (31 g, 87%). Die Substanz wird im Exsiccator im Dunkeln über CaCl₂ getrocknet.
Die getrocknete Verbindung (Carboethoxymethyliden-triphenylphosphoran, 30 g, 0.086 Mol) wird in CH₂Cl₂ (160 mL) gelöst und die Lösung im Eisbad auf 5°C gekühlt. Die Lösung von Brom (13.9 g , 0.087 Mol) in CH₂Cl₂ (40 mL) wird langsam zugetropft und der Ansatz noch 30 min bis zum Verschwinden der Bromfärbung nachgerührt.

Im Scheidetrichter wird die organische Phase zunächst mit Wasser (50 mL), dann zweimal mit NaHCO₃-Lösung (100 mL) gewaschen, bis das gesamte HBr neutralisiert ist. Die CH₂Cl₂ Phase wird über Na₂SO₄ sicc. getrocknet und im Vakuum eingeengt. Der Rückstand wird aus Aceton/n-Hexan (60 mL, 2:1) kristallisiert. Die Kristalle werden mit dieser Mischung (40 mL) gewaschen und im Vakuum getrocknet: 27.5g.
Schmp.: 151.9 °C, Ausbeute: 75 %; C₂₂H₂₀BrO₂P; MW = 427.28.
IR (KBr): 1/λ (cm⁻¹) - 2981 ,1650, 1583, 1434, 1301, 1101, 693; -
¹H-NMR (CDCl₃ :) δ[ppm] = 7.73 - 7.44 (m; 15H, ar.); 3.935 (q; J=7 Hz, 2H); 0.885 (t; J=7 Hz, CH₃).
¹³C-NMR (CDCl₃:) δ[ppm] = 162.5, 149.8, 141.8, 137.9, 123.7, 118.1, 63.2, 14.1.

### c) 2-Brom-3-(4-pyridyl)-propensäureethylester

Unter Lichtausschluss wird Brom-carbethoxymethylen-triphenylphosporan (9.0 g, 21 mMol) in Toluol (60 mL) in Lösung gebracht und anschließend die Lösung von Isonicotin-aldehyd (4-Pyridin-carbaldehyd, 2.14 g, 20 mMol) in Toluol (9 mL) zugesetzt. Der Ansatz wird im Dunkeln 16 h bei RT gerührt. Die Lösung wird daraufhin im Vakuum eingeengt, der Rückstand mit Ether (40 mL) digeriert und die Etherphase vom kristallinen Feststoff abgesaugt. Die Kristalle werden noch 2-mal mit Ether (10 mL) gewaschen und die gesammelten Etherlösungen werden im Vakuum eingeengt. Der verbleibende Rückstand (6.63 g) wird sc mit einem Ether/n-Hexan-Gemisch (2:1) an Al₂O₃ gereinigt. Die gesuchte Substanz erscheint in den Fraktionen 1-5: 4,9 g dunkelbraunes Öl.
Ausbeute: 95.7 %; C₁₀H₁₀BrNO₂; MW = 256.10.; -
¹H-NMR (CDCl₃:) δ[ppm]: 8.72 - 8.69 / 7.66 - 7.63 (AA ' BB' ; 4H, ar.); 8. 12 (s, 1H) ; 4.43 - 4.32 (q, J=7.1Hz, 2H, CH₂) ; 1.44 - 1.37 (t; J=7.1 Hz, CH₃)
¹³C-NMR (CDCl₃ :) δ[ppm]: 162.5,; 149.8,; 141.8,; 137.9,; 123.7, ; 118.1,; 63.2,; 14.1

### d) 3-(4-Fluorphenyl)-2-(4-pyridyl)-6,7-dihydro-5H-pyrrolizin-1-carbonsäureethylester

N-(4-Fluorbenzoyl)-prolin ( 9.48 g, 40 mMol) wird in Aceanhydrid (60 mL) suspendiert, in einer Rückflussapparatur unter Argon auf 80°C temperiert, bis eine klare Lösung erhalten wird (20 min), dann tropfenweise mit 2-Brom-3-(4-pyridyl)-acrylsäureethylester (12.28 g, 8 mMol) in Toluol (10 mL) versetzt (8 min) und das Reaktionsgefäß in ein vorbereitetes heißes Ölbad (120 °C) getaucht. Man erhitzt die dunkle Mischung 21 h unter Rückfluss, worauf dc kein Edukt mehr nachweisbar ist (Al₂O₃; Ethylacetat/n-Hexan 1:1). Die Reaktionsiösung wird abgekühlt und mit Ethylacetat (50 mL) versetzt, der organische Überstand wird abgegossen. Der im Kolben abgeschiedene Feststoff wird 2-mal mit Ethylacetat (30 mL) in der Hitze digeriert. Überstand und Ethylacetat-Lösungen werden vereint und im Vakuum eingeengt. Der Rückstand wird in Ethylacetat (200 mL) aufgenommen und die Ethylacetatphase mit wasser (100 mL), NaHCO₃-Lösung (100 mL) und wieder Wasser (100 ml) neutral gewaschen, getrocknet (Na₂SO₄ sicc) und eingeengt. Aus der eingeengten Ethylacetatphase (30 mL) kristallisiert ein hellbeiger Feststoff. Nach Absaugen, Waschen (Ethylacetat) und nach Trocknen bleiben 2.93 g des gesuchten Produktes.
Nach Einengen der Mutterlauge werden weitere 2.1 g eines Kristallisates erhalten das eine Mischung aus Produkt und Nebenprodukt darstellt.
Ausbeute: 21 %, C₂₁H₁₉FN₂O₂; MW = 350.40.
IR (KBr) : 1/λ (cm⁻¹) = 2985, 1695, 1510, 1222, 1136, 1093, 839, 584, 525
¹H-N_{M}R (CDCl₃:) δ[ppm] = 8.46 - 8.43 /. 7.14 - 7.11 (AA'BB'; 4H, -pyridyl); 7.10 - 6.92 (m; 4H, ar.); 4.22 - 4.12 (q; J=7.1Hz, 2H); 4.02 - 3.95 (t; J=7.2Hz, 2H); 3.27 - 3.19 (t; J=7.5Hz, 2H); 2.63 - 2.48 (m; J=7.3Hz, 2H); 1.24 - 1.17 (t;J=7.2Hz, CH₃)
¹³C-NMR (d₆-DMSO): δ[ppm] = 164.5, 148.9, 145.2, 143.5, 131.1, 131.0, 127.2, 156.0, 115.8, 115.4, 59.4, 46.8, 26.6, 26.4, 14.3.

### Beispiel 1B: 3-(4-Fluorphenyl)-1-(4-pyridyl)-6,7-dihydro-5Hpyrrolizin-2-carbonsäureethylester

SC Trennung des unter Beispiel 1A, d) erhaltenen Gemisch-Kristallisates (2.1 g):
Trennung auf Al₂O₃- Ether /THF 9:1: Fraktionen 6 - 9: Substanz 1A (1.15 g)
Fraktion 10 -12: Gemisch 1A + 1B;
Fraktionen 13 - Ende: Substanz 1B (0.3 g);
Ausbeute: 0.3 g (2 %) C₂₁H₁₉FN₂O₂; MW = 350.40.
IR (KBr) : 1/λ (cm⁻¹) = 2983, 1702, 1600, 1523, 1489, 1435, 1218, 1171, 1161, 1028, 849, 8 34,
¹H-NMR (CDCl₃) δ[ppm] = 8.56 - 8.53 / 7.32 - 7.29 (m; 4H, - pyridyl); 7.48 - 7.41 / 7.16 -7.07 (m; 4H, ar.); 4.09 - 3.99 (q; J=7.1Hz, 2H); 3.92 - 3.85 (t; 7.1Hz, CH₂); 3.03 - 2.95 (t; J=7.3Hz, CH₂); 2.58 - 2.48 (quin; J=5.4Hz, CH₂) ; 1.02 - 0.94 (t; J=7.2 Hz, CH₃)
¹³C-NMR (CDCl₃) : δ[ppm] = 165.1; 163.8; 160.1; 149.2; 143.7; 136.7; 133.0; 131.8; 131.6; 127.8; 123.8; 115.2; 114.8; 59.7; 46.1; 27.0; 24.5; 13.7

### Beispiel 2: 3-(4-Fluorphenyl)-2-(4-pyridyl)-6,7-dihydro-5Hpyrrolizin-1-carbonsäure

3-(4-Fluorphenyl)-2-(4-pyridyl)-6,7-dihydro-5H-pyrrolizin-1carbonsäureethylester (Beispiel 1A , 1,0 g, 2.8 mMol) wird in einer ethanolischen KOH (10 %, 10 mL, 18 mMol) 16 h refluxiert. Anschließend wird der Ethanol im Vakuum abgedampft, der Rückstand in Wasser (10 mL) aufgenommen und mit verd. HCl neutralisiert bis sich die freie Säure vollständig abgeschieden hat. Diese wird abgesaugt, mit Wasser gewaschen und im Vakuum getrocknet. Man erhält 0.78 g.
Schmp.: Zers.; Ausbeute: 85 %; C₁₉H₁₅FN₂O₂; MW = 322.34.
IR (KBr): 1/λ (cm⁻¹) = 1683, 1602, 1512, 1149; -
¹H-NMR (CDCl₃ / d₆-DMSO) δ[ppm] = 8.40 - 8.37 / 7.19 - 7.16 (AA'BB'; 4H, -pyridyl); 7.07 - 6.92 (m; 4H, ar.); 3.98 (t; J=7. 1Hz, CH₂)/ 3.23 (t; J=7.4 Hz, CH₂) 2.92 (s; OH); 2.54 (t; J=7.3Hz, CH₃) .
¹³C-NMR (CDCl₃ / d₆-DMSO) : δ[ppm] = 165.8, 164.2, 159.3,; 148.4,; 145.3,; 143.6, 131.2, 131.0, 127.4, 127.3, 126.5, 126.0, 123.8, 115.6, 115.2, 105.9, 46.6, 26.5, 26.2.

### Beispiel 3: [3-(4-Fluorphenyl)-2-(4-pyridyl)-6,7-dihydro-5Hpyrrolizin-1-yl]-methanol

Unter Argon wird 3-(4-Fluorphenyl)-2-(4-pyridyl)-6,7-dihydro-5H-pyrrolizin-1-carbonsäureethylester (Beispiel 1A , 4.56 g, 13 mMol) in abs. THF (100 mL) gelöst und durch ein Septum über die Kanüle einer Spritze Na-bis-methoxyethoxy-aluminiumhydrid (Vitride^{R}) zugetropft (20 min). Bei 50°C wird 2 h gerührt, worauf dc kein Edukt mehr nachzuweisen ist (Al₂O₃ - Ethylacetat/n-Hexan 3:7), dann lässt man abkühlen. Zur Reaktionslösung wird vorsichtig H₂O (25 mL) getropft, dann im Vakuum eingeengt (THF abgedampft), der wässrig organische Rückstand mit Ethylacetat (100 mL) versetzt. Die Phasen werden getrennt. Die organische Phase wird 2-mal mit Wasser (40 mL) gewaschen, über Na₂SO₄ sicc getrocknet und im Vakuum eingeengt. (5,0 g) Der rotlich braun gefärbte, harzige Rückstand wird aus Diethylether (10 mL) kristallisiert. Die Kristalle werden mit Ethylacetat (5 mL) und Ether (5 mL) gewaschen und getrocknet.
Es verbleiben 3.67 g der gesuchten Verbindung
Ausbeute: 91.5 %; C₁₉H₁₇FN₂O; MW = 308.35;
¹H-NMR (CDCl₃) : δ[ppm] = 8.43 - 8.40 u. 7.21-7.18 (AA'BB'; 4H, - pyridyl); 7.18 - 7.12 (m; 2H, ar.); 7.05 - 6.95 (m; 2H, ar.); 4.55 (s; CH₂); 3.96 (t; J=7.0 Hz, CH₂); 3.01 (t; J=7.3 Hz, CH₂); 2.53 (quin; J=7.1 Hz, CH₂).

### Beispiel 4: 5-(4-Fluorphenyl)-7-methyl-6-(4-pyridyl)-2,3-dihydro-1H-pyrrolizin

(3-(4-Fluorphenyl)-2-(4-pyridyl)-6,7-dihydro-5H-pyrrolizin-1-yl]-methanol (3.08 g, 10 mMol) wird mit Iodwasserstoffsäure (57 %, 18 mL, 134 mMol HI) im Ölbad bei 120°C erhitzt (Reflux). Die zunächst ungelöste Substanz hat sich nach 45 min im Ansatz verteilt, dc (Ether, Al₂O₃) ist kein Edukt (rf=0.05) mehr nachweisbar (Produkt rf=0.9, Iodid rf= 0.6). Die Lösung wird nach dem Abkühlen (1h) mit 50 mL Wasser verdünnt und mit 100 mL Ethylacetat überschichtet. Die Wasserphase wird mit ges. Na₂CO₃-Lsg (30 mL) vorsichtig neutralisiert. und die Phasen getrennt. Die Wasserphase erneut mit Ethylacetat (50 mL) extrahiert, die vereinten Ethylacetatauszüge mit Natriumthiosulfatlösung (Na₂S₂O₃, 2% ig, 40 mL) entfärbt und nach erneutem Waschen mit Wasser (50 mL) über Na₂SO₄ sicc getrocknet. Nach Abdampfen des Lösungsmittels im Vakuum bleiben 2.28 g Rückstand, der aus CH₂Cl₂/Ethylacetat zu einer harzigen Masse erstarrt. Die Substanz wird sc (Al₂O₃/Ether) gereinigt und die aus den Fraktionen durch Abdampfen des Lösungsmittels erhaltene Substanz (Fraktionen 1-17, 1.74 g) aus Diisopropylether kristallisiert. Es werden 1.37 g reiner Verbindung erhalten.
Ausbeute: 47 %; C₁₉H₁₇FN₂; MW = 292.36
¹H-NMR (CDCl₃) δ[ppm] = 8.45 - 8.41 / 7.07 - 7.03 (AA'BB'; 4H, - pyridyl); 7.17 - 6.93 (m; 4H, ar.); 3.945 (t; J=6.9 Hz, CH₂) ; 2.88 (t; J=7.2 Hz, CH₂) ; 2.51 (quin; J=7.0 Hz, CH₂) ; 2.10 (s; CH₃)
¹³C-NMR (CDCl₃) : δ[ppm] = 164.2; 159.2; 149.5; 144.7; 135.5; 130.7; 130.6; 128.6; 128.5; 124.9; 124.6; 122.8; 115.7; 115.3; 108.4; 46.1; 27.2; 23.3; 10.5

### Beispiel 5: 5-(4-Fluorphenyl)-6-(4-pyridyl)-2,3-dihydro-1Hpyrrolizin

3-(4-Fluorphenyl)-2-(4-pyridyl)-6,7-dihydro-5H-pyrrolizin-1carbonsäureethylester (Beispiel 1A, 2.63 g, 7.5 mMol) wird mit Iodwasserstoffsäure (57 %, 7.5 mL, 56 mMol HI) bei RT zunächst gelöst. Das nach wenigen Minuten wieder auskristallisierte Hydroiodid wird durch Erwärmen auf 70°C wieder in Lösung gebracht und die Mischung für 2 h refluxiert. In dieser Zeit, nach ca. 45 min und nach 1 h, werden jeweils 2 weitere Portionen HI (57 %, a 3mL, 45 mMol) zugesetzt. Nach Ablauf der Reaktionszeit ist dc (Al₂O₃, Ether -THF 9:1) kein Edukt (rf=0.4) mehr nachweisbar. Zur Isolierung des Produktes (rf = 0.55) wird der beim Abkühlen gebildete Niederschlag des kristallisierten Produkt-Hydroiodids abgesaugt, durch Abwaschen mit Wasser (3-mal 10 mL) von anhaftender HI gereinigt und getrocknet. An Hydroiodid werden 2.57 g (84.3 %) isoliert.
Isolierung der Base:
5- (4-Fluorphenyl)-6-(4-pyridyl)-2, 3-dihydro-1H-pyrrolizinhydroiodid (3.68 g, 9 mMol) wird in CH₂Cl₂ (150 mL) suspendiert und mit gesättigter NaHCO₃-Lösung (100 mL) intensiv gerührt. Die CH₂Cl₂-Phase wird anschließend abgetrennt, mit Na₂SO₄ sicc getrocknet und im Vakuum eingeengt. Der Rückstand wird in wenig Ether aufgenommen und die gebildeten Kristalle werden abgesaugt und getrocknet: 2.39 g.
Ausbeute: 80 %, C₁₈H₁₅FN₂; MW = 278.33
IR (KBr): 1/λ (cm⁻¹) = 1594, 1525, 1508, 1426, 1356, 1216, 833, 787; -
¹H-NMR (CDCl₃) δ[ppm] = 8.38 - 8.35 / 7.11 - 7.09 (AA'BB'; 4H, - pyridyl); 7.31 - 7.24 (m; 2H, ar.); 7.11 - 7.02 (m; 2H, ar.); 6.15 (s; 1H); 3.89 (t; J=7.0Hz, CH₂) ; 2.945 (t; J=7.2Hz, CH₂); 2.51 (quin; J=7.0Hz, CH₂); -
¹³C-NMR (CDCl₃): δ[ppm] = 160.0, 157.0, 149.2, 145.0, 137.7, 131.2, 131.1, 128.8, 122.0, 116.1, 115.6, 99.8, 45.9, 27.4, 24.5

### Beispiel 6A: 3-(2-thienyl)-2-(4-pyridyl)-6,7-dihydro-5H-pyrrolizin-1-carbonsäureethylester

### a) N-(2-thienyl-carbonyl)-prolin

L-Prolin (2.42 g, 21 mMol) wird in NaOH (5 %, 30 mL, 38 mMol) gelöst und die Lösung im Eisbad abgekühlt (5°C). Unter Rühren wird Thiophen-2-carbonsäurechlorid (2.93 g, 20 mMol) langsam (10-15 min) zugetropft, wobei die Temperatur 7°C nicht überschreiten sollte. Die Mischung wird 1h bei 5-7°C gerührt, die Kühlung entfernt und 1 h bei RT weitergerührt. Nach Ansäuern der Reaktionsmischung mit HCl (10%ig, 12 mL) setzt sich das Amid ölig ab. Das Öl wird mit Ethylacetat (300 mL) extrahiert, die Ethylacetatphase mehrmals mit Wasser (200 mL) gewaschen, getrocknet (Na₂SO₄ sicc.) und das Lösemittel im Vakuum abgedampft. Der Rückstand (4.16 g) wird mit heißem Wasser (20 mL) zweimal aufgeschlämmt, abgesaugt und mit Diisopropylether (10 mL) gewaschen. Die Substanz wird im Vakuum über P₂O₅ getrocknet (dc: RP 18 / MeOH, rf=0.75): 3.87 g.
Schmp.: 143.0°C, Ausbeute: 81.6%; C₁₂H₁₂FNO₃; MW = 237.23.
IR (KBr): 1/λ (cm⁻¹) = 3091, 3078, 1717, 1601, 1524, 1439, 1406, 1262, 1240, 1195, 773, 757, 736; -
¹H-NMR (CDCl₃ :) δ[ppm] = 7.70 u. 7.68 (d; J=3.7Hz, 1H) 7.62 u. 7.59 (d; J=5.0Hz, 1H 7.16 -7.12 (dd; 1H) 4.85-4.79 (m; 1H) 3.94-3.87 (m; CH2) 2.50 (m; 1H) 2.12 (m; 3H);

### b) 3-(2-thienyl)-2-(4-pyridyl)-6,7-dihydro-5H-pyrrolizin-1carbonsäureethylester

N-(2-thienyl-carbonyl)-prolin (4.69 g, 21 mMol) wird in Acetanhydrid (15 mL) bei 60°C gelöst (15 min), zur klaren Lösung wird 2-Brom-3-(4-pyridyl)-propensäureethylester (6.40 g, 25 mMol) zugegeben und die Mischung bei 90°C 16 h erwärmt, worauf eine dc Probe (Al₂O₃-Ethylacetat/THF 9:1) kein Edukt mehr anzeigt. Die abgekühlte Reaktionsmischung wird mit Ethylacetat (120 mL) versetzt und mit gesättigter Na₂CO₃-Lösung (80 mL) 15 min intensiv gerührt und mit Wasser (50 mL) verdünnt. Die Ethylacetatphase wird abgetrennt, die Wasserphase mit weiteren 3 Portionen Ethylacetat (150 mL) extrahiert, diese organischen Phasen vereint, mit gesättigter Na₂CO₃-Lsg (50 mL) gewaschen und nach Trocknen (K₂CO₃ sicc.) im Vakuum eingeengt. Als Rückstand werden 8 g erhalten, die in wenig Ethylacetat gelöst und sc (Al₂O₃-Ethylacetat/n-Hexan 2:1) gereinigt werden.
Fraktionen 6-12: enthalten die gesuchte Substanz , die aus Ether/Diisopropylether kristallisiert, abgesaugt und getrocknet wird: 2,63 g.
Ausbeute: 37 %, C₁₉H₁₈N₂O₂S, MW = 338.43;
¹H-NMR (CDCl₃ :) δ[ppm] = 8.52 - 8.49 / 7.23 - 7.20 (AA 'BB' ; 4H, -pyridyl u. 1H; -thiophen); 6.925 (dd; J=3.6Hz, 1H); 6.75 (d; J=3.5Hz, 1H); 4.20 - 4.07 (m; 4H, CH₂); 3.23 (t; J=7.5Hz, CH₂); 2.575 (quin; J=7.3Hz, CH₂); 1.173 (t; J=7.1Hz, CH₃)
Fraktionen 13-15: Mischfraktion mit Produkt aus Beispiel 6B: 0.23 g

### Beispiel 6B: 3-(2-thienyl)-1-(4-pyridyl)-6,7-dihydro-5Hpyrrolizin-2-carbonsäureethylester

Aus SC-Reinigung des Beispiel 6A (Al₂O₃-Ethylacetat/n-Hexan 2:1):
Fraktionen 19-30: enthält die stellungsisomere Verbindung, die ebenfalls aus Ether/Diisopropylether rein kristallisiert, abgesaugt und getrocknet wird: 0,19 g.
Fraktionen 31 - Ende: enthält Verbindung 6B verunreinigt mit N-(2-thienyl-carbonyl)-prolin.
Ausbeute: 2.7 %, C₁₉H₁₈N₂O₂S, MW = 338.43;
¹H-NMR (CDCl₃:) δ[ppm] = 8.56 - 8.53 (AA ' BB ' ; 2H, -pyridyl); 7.43 - 7.40 (d; J=3.1Hz, 1H, -thiophen); 7.30 - 7.29 (m; 3H, - pyridyl u.-thiophen); 7.12 - 7.08 (dd; J=3.7 Hz, 1H); 4.15 - 3.99 (m; 4H, CH₂); 2.99 (t; J=7.3 Hz, CH₂); 2.53 (quin; J=7.2Hz, CH₂); 1.035 (t; J=7.2 Hz, CH₃) .

### Beispiel 7: 3-(2-Thienyl)-2-(4-pyridyl)-6,7-dihydro-5Hpyrrolizin-1-carbonsäure

### analog zu Beispiel 2:

3-(2-Thienyl)-2-(4-pyridyl)-6,7-dihydro-5H-pyrrolizin-1-carbonsäureethylester (Beispiel 6A, 1.5 g, 4.4 mMol) wird in einer ethanolischen KOH (8 %, 15 mL, 21 mMol) 16 h bei 60°C gerührt, der Ethanol im Vakuum abgedampft, der Rückstand in Wasser (40 mL) aufgeschlämmt und im Eisbad mit verd. H₃PO₄ (8%) auf pH4 gebracht. Der gebildete rotbraune Niederschlag wird abgesaugt, mit Wasser gewaschen und im Vakuum getrocknet. Man erhält 1.17 g.
Schmp.: Zers.; . Ausbeute: 85 %; C₁₉H₁₅FN₂O₂; MW = 322.34.
IR (KBr): 1/λ (cm⁻¹) = 1676, 1604, 1567, 1420, 1305. 1196, 1012, 852, 702; -
¹H-NMR (CDCl₃) δ[ppm] = 8.49 - 8.46 / 7.53 - 7.50 (AA'BB'; 4H, - pyridyl); 7.335 (d; J=3.1Hz, 1H, -thiophen); 7.00 (d; J=3.7Hz, 1H); 6.81 (dd, 1H, -thiophen); 4.79 (s, OH) 4.07 (t; J=7.1 Hz, CH₂); 3.28 (t; J=7.3Hz, CH₂) ; 2.585 (m; CH₂).

### Beispiel 8: 5-(2-Thienyl)-6-(4-pyridyl)-2,3-dihydro-1Hpyrrolizin

3-(2-Thienyl)-2-(4-pyridyl)-6,7-dihydro-5H-pyrrolizin-1carbonsäure (Beispiel 7, 0.2 g, 0.64 mMol) wird unter Argon in einem 10 mL Kolben durch Eintauchen in ein Metallbad auf 250°C erhitzt. Die CO₂-Entwicklung erfolgt sofort, die Substanz schmilzt auf und verfärbt sich schwarz. Nach dem Abkühlen wird die erstarrte, glasartige Schmelze in CH₂Cl₂ (10 mL) aufgenommen und über Al₂O₃ (TSC, Baker) filtriert. Der Filterrückstand wird mit Diethylether eluiert. Das Eluat wird eingeengt und aus Ether / Diisopropylether in der Kälte kristallisiert: weiße Kristalle 0.04 g.
Ausbeute: 23 %; C₁₆H₁₄N₂S; MW = 266.37.
¹H-NMR (CDCl₃) δ[ppm] = 8.44 - 8.41 / 7.27 - 7.24 (AA'BB'; 4H, - pyridyl); 7.385 (dd, 1H, -thiophen); 7.07 (d; 2H, -thiophen); 6.99-6.97 (d; 1H, -thiophen); 6.16 (s; 1H); 3.955 (t; J=7.1 Hz, CH₂); 2.94 (t; J= 7.3 Hz, CH₂); 2.52 (quin; J=7.2 Hz, CH₂).

### Beispiel 9A: 3-(5-Chlor-2-thienyl)-2-(4-pyridyl)-6,7-dihydro-5H-pyrrolizin-1-carbonsäureethylester

### a) (5-Chlor-2-thienyl-carbonsäurechlorid

5-Chlor-thiophen-2-carbonsäure (4.0 g, 24.6 mMol) wird in Dichlorethan (10 mL) gelöst und Thionylchlorid (SOCl₂, 2,7 mL, 4.4 g, 37 mMol) zugegeben, nach Impfen mit einem Tropfen DMF wird der Ansatz auf 50°C (IT) erwärmt. Der Verlauf der Umsetzung wird dc (SiO₂ / Ether: Produkt rf 0.8, Edukt rf 0.1-0.3) verfolgt. Nach 1,5 h und nach 2,5 h wurde jeweils SOCl₂ (5 Tropfen) zugefügt. Nach 4.5 h wird die hellgelbe Lösung am Rotavapor eingeengt und der Rückstand fraktionierend destilliert: bei 50 mbar wird bei der Übergangstemperatur 110°C 4.0 g Produkt (89.9 %) erhalten.
Ausbeute: 4.0 g ( 90%); C₅H₂Cl₂OS; MW = 181.04.
¹H-NMR (CDCl₃:) δ[ppm] = 7.80 / 7.05 (AB; 2H, J_{(AB)} = 4 Hz)

### b) N-(5-Chlor-2-thienyl-carbonyl)-prolin

Zu einer im Eisbad auf 5°C gekühlten Lösung von L-Prolin (2.42 g, 21 mMol) in NaOH (5 %, 32 mL, 40 mMol) wird unter Rühren 5-Chlor-thiophen-2-carbonsäurechlorid (3.62 g, 20 mMol) langsam (10-15 min) zugetropft, wobei die Temperatur 9°C nicht überschreitet. Die Mischung wird 30 min bei 5-7°C gerührt, dabei bildet sich langsam ein Niederschlag der durch Zugabe von Wasser (8 mL) wieder in Lösung gebracht wird. Der Ansatz wird noch 90 min bei RT gerührt. Nach Ansäuern der Reaktionsmischung mit HCl (10%ig, 12 mL, pH 3.5) setzt sich das Amid nach 30 min Rühren kristallin ab. Die Kristalle werden abgesaugt, mehrmals mit Wasser (200 mL) gewaschen und getrocknet: Kristallisat 1 = 2.61 g.

Die Mutterlauge wird mit HCl auf pH 1-2 angesäuert und der gebildete voluminöse Niederschlag ebenfalls abgesaugt und mit Wasser neutral gewaschen und getrocknet: Kristallisat 2: 2.17 g.
Beide Kristallisate stellen die gesuchte Substanz dar: Gesamtausbeute 4.78 g.
Ausbeute: 92%; C₁₀H₁₀ClNO₃S; MW = 259.71.
¹H-NMR (CDCl₃:) δ[ppm] = 8.50 (s; OH) 7.43 / 6.94 (AB; 2H, - thiophen, J_{(AB)} = 4 Hz); 4.77 - 4.72 (m; 1H) ; 3.91 - 3.77 (m; CH₂); 2.37 - 2.12 (m; 4H, CH₂) .

### c) 3-(5-Chlor-2-thienyl)-2-(4-pyridyl)-6,7-dihydro-5H-pyrrolizin-1-carbonsäureethylester

N-(5-Chlor-2-thienyl-carbonyl)-prolin (1.1 g, 4.2 mMol) wird in Acetanhydrid (3 mL) bei 50°C (IT) gelöst (15 min), zur klaren Lösung wird 2-Brom-3-(4-pyridyl)-propensäureethylester (1.29 g, 5.0 mMol zugegeben und die Mischung bei 90°C 22 h erwärmt, worauf eine dc Probe (Al₂O₃-Ethylacetat/THF 9:1) kein Edukt mehr anzeigt.
Die abgekühlte Reaktionsmischung wird mit Ethylacetat (50 mL) und Wasser (50 mL) versetzt und 15 min intensiv gerührt. Die Phasen werden getrennt, die Wasserphase wird zur Verbesserung der Phasentrennung mit gesättigter NaCl-Lösung (50 mL) versetzt und mit weiteren 3 Portionen Ethylacetat (150 mL) extrahiert.
Danach wird die Wasserphase mit gesättigter Na₂CO₃-Lsg (50 mL) auf pH 7-8 abgestumpft und nochmals mit Ethylacetat (50 mL) extrahiert.
Die Ethylacetatphasen werden gesammelt und vereinigt, mit gesättigter Na₂CO₃-Lsg (50 mL) und Wasser (50 mL) gewaschen und nach Trocknen (K₂CO₃ sicc.) im Vakuum eingeengt. Als Rückstand werden 1.2 g erhalten, die in wenig THF gelöst und sc (Al₂O₃ (300g)-Ether/THF 9:1) gereinigt werden.
Fraktionen 1-30: enthalten die gesuchte Substanz, die aus Ether/Diisopropylether kristallisiert, abgesaugt und getrocknet wird: 0,6 g.
Ausbeute: 38 %, C₁₉H₁₇ClN₂O₂S, MW = 372.88;
¹H-NMR (CDCl₃ :) δ[ppm] = 8.54 - 8.51 / 7.30 - 7.28 (AA'BB'; 4H, -pyridyl) ; 6.75 / 6.53 (AB; 2H, -thiophen, J_{(AB)} = 4 Hz); 4.20 - 4.04 (m, 4H, CH₂); 3.22 (t; J=7.5Hz, CH₂) 2.59 (quin; J=7.3Hz, CH₂) ; 1.17 (t; J=7.1Hz, CH₃) .

### Beispiel 9B: 3-(5-Chlor-2-thienyl)-1-(4-pyridyl)-6,7-dihydro-5H-pyrrolizin-2-carbonsäureethylester

Aus SC-Reinigung des Beispiel 9A (Al₂O₃-Ether/THF 9:1):
Fraktionen 35 - 60: enthält die stellungsisomere Verbindung, die ebenfalls aus Ether/Diisopropylether rein kristallisiert, abgesaugt und getrocknet wird: 0.11 g.
Ausbeute: 1.5 %, C₁₉H₁₇ClN₂O₂S, MW = 372.88;
¹H-NMR (CDCl₃: ) δ[ppm] = 8.56 - 8.53 / 7.34 - 7.31(AA'BB'; 4H, - pyridyl); 7.03 / 6.91 (AB; 2H, -thiophen, J_{(AB)} = 4 Hz); 4.17 - 3.99 (q + t; 4H, CH₂) ; 3.00 (t; J=7.3Hz, CH₂) ; 2.545 (quin; J=7.2Hz, CH₂); 1.07 (t; J=7.1Hz, CH₃) .

### Beispiel 10A: 5-(4-Fluorphenyl)-6-(4-pyridyl)-2,3-dihydropyrrolo-[2,1-b]-thiazol-7-carbonsäureethylester

### a) Thiazolidin-2-carbonsäure-ethylester

Glyoxalsäure-ethylester (50% in Toluol, 40.92 g, 0.2 Mol), verdünnt mit Toluol (15 mL), wird auf 5°C gekühlt, Cysteaminhydrochlorid (22.72 g, 0.2 Mol) wird zugegeben und eine gesättigte NaHCO₃-Lösung (8.7 % ig, 120 mL) langsam zugetropft (2 h). Die Temperatur wird während des Zutropfens unter 15°C gehalten. Das 2-Phasensystem wird über 16 h bei RT gerührt.
Die Reaktion wird mittels GC kontrolliert: HP17, 10 m, 0.53mm; Temp.: 120°C (0.5 min), 20°C / min, 180 °C (1 min), Inj 220°C, Det. 280°C; rt 2.57.
Danach wird die Toluolphase abgetrennt, die wässrige Phase (pH 7-8) mit Ether (150 mL) extrahiert. Toluolphase und Etherphase werden über Na₂SO₄ (sicc.) getrocknet und eingeengt und die öligen Rückstände vereinigt: 26.9 g.
Ausbeute (roh): 83.5 %, C₆H₁₁O₂S, 161.22 g/mol;
IR (KBr): 1/λ (cm⁻¹) = 3303, 2980, 2939, 1733, 1672, 1516, 1443, 1369, 1282, 1182, 1026; -
¹H-NMR (CDCl₃:) δ[ppm] = 4.92 (s,1H); 4.25 (q, 2H, J=7.1Hz); 3.66 (m, 1H); 3.10 - 2.95 (m, 2H); 2.90 - 2.78 (m, 1H); 2,44 (s, OH ); 1.305 (t, 3H, J=7.1Hz)

### b) 3-(4-Fluorbenzoyl)-thiazolidin-carbonsäure-ethylester

Die Lösung von Thiazolidin-2-carbonsäure-ethylester (24.18 g, 0.15 Mol) in abs. CH₂Cl₂ (80 mL) wird bei RT tropfenweise mit der Lösung von 4-Fluorbenzoylchlorid (23.78, 0.15 Mol) in abs. CH₂Cl₂ (20 mL) versetzt. Die Lösung erwärmt sich auf 38°C, das Lösemittel beginnt zu sieden und HCl wird freigesetzt. Unter Erwärmen wird 4 h am Rückfluss gehalten.
Die CH₂Cl₂-Phase wird mit Na₂CO₃-Lsg (100 mL) ausgerührt (30 min) mit Wasser (50 mL) gewaschen und abgetrennt. Sie wird über Na₂SO₄ (sicc.) getrocknet und eingeengt. Der verbleibende Rückstand wird aus Diisopropylether (30 mL) kristallisiert, die Kristalle mit Diisopropylether (25 mL) gewaschen und getrocknet: 34.07 g.
Ausbeute: 80.2 %, C₁₃H₁₄FNO₃S, MW = 283.32;
¹H-NMR (CDCl₃ :) δ[ppm] = 7.52 (m; 2H, ar.); 7.17 - 7.05 (m; 2H, ar.); 4.30 - 4.20 (q; J=7.1 Hz, CH₂) ; 3.98-3.87 (m; CH₂) ; 3.32 - 3.19 (m; 1H); 3.09 - 2.98 (m; 1H); 1.305 (t; J=7.1 Hz, CH₃).

### c) 3-(4-Fluorbenzoyl)-thiazolidin-carbonsäure

3-(4-Fluorbenzoyl)-thiazolidin-carbonsäure-ethylester (28.33 g, 0.1 Mol) wird in ethanolischer KOH (5 % ig, 165 mL, 0.15 Mol) suspendiert und die Mischung anschließend für 2 h auf 50°C (IT) erhitzt. Die abgekühlte Lösung wird mit Wasser (100 mL) und verd. Phosphorsäure (8%, 150 mL) angesäuert (pH 2-3). Ausgefallene Salze werden durch Zugabe von Wasser (150 mL) wieder in Lösung gebracht, Diese wässrige Lösung wird mit Diethylether (500 mL) extrahiert, die etherischen Extrakte werden mit ges. NaCl-Lösung (100 mL) gewaschen, über Na₂SO₄ (sicc.) getrocknet und im Vakuum eingeengt. Der Rückstand wird aus Diisopropylether (40 mL) kristallisiert: 24.87 g.
Ausbeute: 97.5 %, C₁₁H₁₀FNO₃S, MW = 255.27;
¹H-NMR (CDCl₃:) δ[ppm] = 8.64 (s; 1H, OH); 7.59 - 7.53 (m; 2H, ar.); 7.19 - 7.09 (m; 2H, ar.); 5.64 (s; CH); 3.98 - 3.92 (m; CH₂) ; 3.36 - 3.24 (m; CH₂); 3.11-3.01 (m; CH₂).

### d) 5-(4-Fluorphenyl)-6-(4-pyridyl)-2,3-dihydro-pyrrolo-[2,1-b]-thiazol-7-carbonsäureethylester

Die bei 90°C erhaltene klare Lösung von 3-(4-Fluorbenzoyl)-thiazolidin-carbonsäure (12.76 g, 0.05 Mol) in Acetanhydrid (60 mL) wird mit 2-Brom-3-(4-pyridyl)-propensäureethylester (13.8 g, 54 mMol) versetzt und 4 d bei 80°C (IT) gerührt. Die schwarz gefärbte Reaktionsmischung wird im Vakuum eingeengt und nach Abkühlen mit MeOH (10 mL) versetzt. Nach vollständiger Reaktion restlichen Acetanhydrids wird nochmals im Vakuum eingeengt und der Rückstand (30 g) in der Wärme in MeOH (7 mL) vollständig gelöst. Bei RT kristallisiert Produkt 10 A (DC: Al₂O₃, Ethylacetat/n-Hexan 6:4; rf = 0.45) aus. Der Kristallbrei wird abgesaugt, die Kristalle gesammelt (2.6 g) und die Mutterlauge zur Gewinnung einer weiteren Kristallfraktion (2.16 g) kalt gestellt (0°C): 4.42 g (24 %)
Die Mutterlauge wird nach Filtration mit CH₂Cl₂ (20 mL) verdünnt und über wenig Al₂O₃ filtriert. Das Al₂O₃ wird mit CH₂Cl₂ (150 mL) nachgewaschen und die Eluate eingeengt: 5.78 g (Mischung aus Produkt A, rf = 0.45 und B, rf = 0.5).
Die gelb gefärbten Kristalle der beiden Kristallfraktionen werden mit wenig kaltem MeOH gewaschen und getrocknet:
Ausbeute: 24 %, C₂₀H₁₇FN₂O₂S, MW = 368.43;
IR (KBr): 1/λ (cm⁻¹) =3066, 2992, 2786, 1722, 1692, 1627, 1520, 1377, 1226, 1147, 820, 596; -
¹H-NMR (CDCl₃:) δ[ppm] = 8.57 - 8.55 / 7.33 - 7.30 (AA'BB'; 4H, -pyridyl); 7.47 - 7.40 (m; 2H, ar.); 7.18 - 7.10 (m; 2H, ar.); 4.11 - 3.99 (q + t; 4H, CH₂); 3.69 (t; J=7.0 Hz, CH₂); 0.98 (t; J=7.1 Hz, CH₃) .

### Beispiel 10B: 5-(4-Fluorphenyl)-7-(4-pyridyl)-2,3-dihydropyrrolo-[2,1-b]-thiazol-6-carbonsäureethylester

Aus der unter Beispiel 10 A erhaltenen Mischung (5.78 g) aus Produkt A (rf = 0.45) und B (rf = 0.5) werden durch SC an Al₂O₃ mit dem Eluenten Ethylacetat/n-Hexan (6:4) wenige Fraktionen reinen Produktes 10 B erhalten:
C₂₀H₁₇FN₂O₂S, MW = 368.43;
¹H-NMR (CDCl₃ :) δ[ppm] = 8.44 - 8.41 / 7.36 - 7.33 (AA'BB'; 4H, ar.); 7.11 - 7.00 (m; 4H, ar.); 4.28 - 4.15 (m; 4H); 3.77 - 3.70 (m; 2H); 1.275 (t; CH₃, J=7.1 Hz).

### Beispiel 11: 5-(4-Fluorphenyl)-6-(4-pyridyl)-2,3-dihydropyrrolo-[2,1-b]-thiazol-7-carbonsäure

Die Verbindung des Beispieles 10 A (1.0 g, 2.7 mMol) wird durch Kochen unter Rückfluss in ethanolischer KOH (10 %, 10 mL, 18 mMol) in 4 h vollständig gespalten (dc). Die ethanolische Lauge wird im Vakuum eingeengt, der salzartige Rückstand in Wasser (40 mL) aufgenommen und der Alkaliüberschuss mit H₃PO₄ (8 %) soweit neutralisiert (pH 4-5), dass die Suspension keine gallertige Konsistenz annimmt. Der Feststoff wird abgesaugt, mit Wasser gewaschen und 24 h im evakuierten Exsiccator über P₂O₅ getrocknet: 0.87 g hellgelbes Pulver
Schmp. 210.5 °C, Ausbeute: 94.6 %; C₁₈H₁₃FN₂O₂S, MW = 340.38;
IR (KBr) : 1/λ (cm⁻¹) = 3399, 1672, 1599, 1534, 1494, 1228, 1160. -
¹H-NMR (d₆-DMSO / CDCl₃) : δ[ppm] = 8.55 - 8.52 / 7.91 - 7.87 (AA'BB'; 2H, -pyridyl); 7.50 - 7.43 (m, 2H); 7.19 - 7.10 (m, 2H); 4.14 (t; J=7.1Hz, CH₂) ; 3.83 (t; J=7.2Hz, CH₂) ; -
¹³C-NMR (d₆-DMSO / CDCl₃) : δ[ppm] = 165.3, 164.6, 159.7, 148.5, 142.7, 140.2, 134.6, 131.5, 131.4, 130.5, 127.22, 127.15, 122.8, 114.8, 114.4, 47.3, 34.4.

### Beispiel 12: [5-(4-Fluorphenyl)-6-(4-pyridyl)-2,3-dihydropyrrolo-[2,1-b]-thiazol-7-yl]-methanol

Die Verbindung des Beispieles 10 A (0.37 g, 1.0 mMol) wird in abs. THF (7 mL) bei 50°C gelöst und mit der Lösung von Na-bis-methoxyethoxy-aluminiumhydrid in Toluol (Vitride^{R}, 70%ig, 0.80 mL, 2.8 mMol) 7 h bei 55°C gehalten. Der Hydridüberschuss wird nach dem Abkühlen im Eisbad mit wenig Wasser zersetzt und die Suspension bis zur vollständigen Bildung des Niederschlages gerührt. Der Niederschlag wird abgesaugt, mit Wasser bis zur neutralen Reaktion der Waschphase gewaschen: 0.27 g.
Ausbeute: 82 %, C₁₈H₁₅FN₂OS, MW = 326.40;
¹H-NMR (DMSO-d₆/ CDCl₃) : δ[ppm] = 8.76 - 8.55 / 7.59 - 7.55 (AA'BB'; 4H, -pyridyl); 7.51 - 7.44 (m; 2H, ar.); 7.20 - 7.11 (m; 2H, ar.); 4.49 (s; CH₂) ; 4.17 (t; J=7.0 Hz, CH₂) ; 3.73 (t; J=7.0 Hz, CH₂) .

### Beispiel 13: 5-(4-Fluorphenyl)-7-methyl-6-pyridin-4-yl-2,3-dihydro-pyrrolo-[2,1-b]-thiazol

Die Verbindung des Beispiels 12 (2.05 g, 6.3 mMol) wird in einer Mischung aus abs. Acetonitril (16 mL), Eisessig (1.6 mL) und wenig abs. DMF bei 50°C in Lösung gebracht, Natriumiodid (2.35 g, 15.7 mMol) wird zugegeben und anschließend Trimethylsilylchlorid (TMSCl, 1. 6mL, 1.37 g, 12.7 mMol) zugetropft. Die Temperatur des Ansatzes wird 2 h bei 50°C gehalten. Es wird
Eisessig (1.6 mL) und Zinkpulver (2.87 g, 44 mMol) in drei Portionen zugesetzt und 16 h bei 80°C gerührt. Die Mischung wird abgekühlt, mit wenig Wasser versetzt und mit CH₂Cl₂ extrahiert. Die CH₂Cl₂ Extrakte werden über Na₂SO₄ sicc. getrocknet und eingeengt. Der erhaltene Rückstand wird in THF aufgenommen und mit THF über Al₂O₃ (50 g) eluiert. Aus dem nach Abdampfen des THF erhaltenen Rückstand (1.4 g) wird mit Aceton 0.6 g hellgelbe Substanz (94 %; HPLC: RP 18; Acetonitril / NaH₂PO₄ Puffer 70:30) erhalten, die aus MeOH in der Wärme umkristallisiert wird: 0.43 g hellgelbes Pulver.
Schmp. 208 °C; Ausbeute: 23 % , C₁₈H₁₅FN₂S, MW = 310.4;
IR (KBr) : 1/λ (cm⁻¹) = 1596, 1557, 1533, 1499, 1420, 1361, 1303, 1226, 1158, 1098, 1053, 989, 852, 822, 735, 721, 529 ; -
¹H-NMR (CDCl₃) : δ[ppm] = 8.57 (2H,-pyridyl); 7.35 - 7.27(m; 4H,pyridyl u. ar.); 7.19 - 7.10 (m; 2H, ar.); 4.12 (t; J=7.0 Hz, CH₂); 3.72 (t; J=7.0 Hz, CH₂); 2.23 (s; CH₃) .
¹³CNMR (d₆-DMSO / CDCl₃) : δ[ppm] = 164.6, 159.7, 147.7, 145.2, 132.6, 131.0, 130.8, 129.8, 127.5, 121.4, 119.4, 116.0, 115.6, 113.3, 47.9, 35.3, 12.8.

### Beispiel 14: 5-(4-Fluorphenyl)-6-pyridin-4-yl-2,3-dihydropyrrolo-[2,1-b]-thiazol

5-(4-Fluorphenyl)-6-(4-pyridyl)-2,3-dihydro-pyrrolo-[2,1-b]-thiazol-7-carbonsäure (Beispiel 11, 120 mg, 0.35 mMol) wir unter Argon in einem Ölbad zunächst 2 h bei 140°C und anschließend 3 h bei 180°C erhitzt. Der braunrote Schmelzkuchen wird nach Abkühlen auf RT mit wenig EtOH digeriert. Die ungelöste, rotbraune Substanz wird abgesaugt, mit Ether gewaschen und getrocknet: 0.07 g.
Ausbeute: 67 %, C₁₇H₁₃FN₂S, MW = 296.37;
¹H-NMR (CDCl₃) : δ[ppm] = 8.46 - 8.43 / 7.64 - 7.61 (AA'BB'; 4H, -pyridyl); 7.44 - 7.37 (m; 2H, ar.); 7.20 - 7.11 (m; 2H, ar.); 6.67 (s; CH); 4.35 (t; J=7.2 Hz, CH₂) ; 3.95 (t; J=7.2 Hz, CH₂).

### Beispiel 15A: 3-(4-Fluorphenyl)-2-(4-pyridyl)-5,6,7,8-tetrahydro-indolizin-1-carbonsäure-ethylester

### a) 1-(4-Fluorbenzoyl)-piperidin-2-carbonsäure

D,L-Pipecolinsäure (16.15 g, 0.125 Mol), gelöst in NaOH (5 %, 200 mL, 0.25 Mol) wird bei 5°C (Eisbadkühlung) tropfenweise mit 4-Fluorbenzoesäurechlorid (19.83 g, 0.125 Mol) versetzt, der sich nach 1 h bildende Brei wird vor weiterer Säurechlorid-Zugabe mit Wasser (50 mL) verflüssigt. Der Ansatz wird 4 h bei 10-15°C gerührt.
Durch Zugabe von Wasser (220 mL) wird der gebildete Niederschlag fast vollständig in Lösung gebracht (pH = 8 - 9) und mit Ether (160 mL) werden die Reste des Säurechlorids und anderer neutraler Verunreinigungen entfernt. Aus der alkalischen Wasserphase wird durch Zugabe von HCl (10%ig) das gewünschte Produkt abgeschieden bis pH 2 erreicht ist. Die HCl-saure Suspension wird mit Ether (300 mL) extrahiert, die Etherphase abgetrennt, getrocknet (Na₂SO₄ sicc.) und im Vakuum eingeengt. Der Rückstand (8% 4-Fluorbenzoesäure) wird aus Ether (20 mL) kristallisiert: 28.67 g.
Ausbeute: 72 %, C₁₃H₁₄FNO₃, MW = 251.3;
¹H-NMR (CDCl₃): δ[ppm] = 8.30 ( br; 1H, OH); 7.50 - 7.35 (m; 2H, ar.); 7.20 - 7.00 (m; 2H, ar.); 5-50 (m; 1H, CH_{X}N); 4.70 - 4.4 (m; 1H, CH_{A}H_{B}) ; 3.75 - 3.60 (m; 1H, CH_{A}H_{B}) ; 3.35 - 3.15 (m; 1H, CH_{A}H_{B}) ; 2.45 - 2.30 (m; 1H, CH_{A}H_{B}) ; 1.9 - 1.3 (m; 4H, CH_{A}H_{B}) .

### b) 3-(4-Fluorphenyl)-2-(4-pyridyl)-5,6,7,8-tetrahydroindolizin-1-carbonsäure-ethylester

1-(4-Fluorbenzoyl)-piperidin-2-carbonsäure (17.6 g, 0.07 Mol)wird bei 80°C in Acetanhydrid (45 mL) gelöst und anschließend 2-Brom-3-(4-pyridyl)-propensäureethylester (22.4 g, 0.087 Mol). Eine starke CO₂-Entwicklung ist nach ca 10 min beendet. Die Reaktionsmischung wird 20 h bei 130°C (Reflux) gehalten. Nach dem Abkühlen werden CH₂Cl₂ (100 mL) und Wasser (50 mL) zugegeben und 10 min kräftig gerührt, anschließend die Phasen getrennt. Die Wasserphase wird nochmals mit CH₂Cl₂ (150 mL) extrahiert, die vereinigten organischen Phasen werden mit ges. Na₂CO₃-Lsg (50 mL) neutral gewaschen, getrocknet (Na_{z}SO₄ sicc.) und eingeengt. Es verbleiben 29.26 g Rohprodukt. Die saure Wasserphase wird mit NaOH alkalisiert, wobei sich schwarze Klumpen absetzen, die in CH₂Cl₂ (200 mL) teils löslich sind und Reste der gesuchten Verbindung enthalten.
Rückstand dieser CH₂Cl₂-Phase und Rohproduktfraktion werden vereinigt (30 g) in CH₂Cl₂ (30 mL) aufgenommen und sc an Al₂O₃ (2300 g) mit Ether / THF (9:1) gereinigt:
Fraktionen 4-12: 12.5 g Produkt 15 A, das aus Diisopropylether/n-Hexan (10 mL, 1:1) kristallisiert. Die Kristalle werden abgesaugt und getrocknet: 9.3 g (36.6%).
Ausbeute: 9.3 g (36.6 %) C₂₂H₂₁FN₂O₂; MW = 364.42.
¹H-NMR (CDCl₃) δ[ppm]: 8.39 - 8.36 / 7.04 - 7.02 (AA'BB'; 4H, - pyridyl); 7.13 - 6.93 (m; 4H, ar.); 4.12 (q; J=7.11 Hz, CH₂); 3.705 (t; J=3 Hz, CH₂); 3.21 (t; J=2 Hz, CH₂); 1.98-1.85 (m, 2 CH₂, 4H); 1.09 (t; J=7.1 Hz, CH₃.)

### Beispiel 15B: 3-(4-Fluorphenyl)-1-(4-pyridyl)-5,6,7,8-tetrahydro-indolizin-2-carbonsäure-ethylester

### SC Reinigung der Verbindung aus Beispiel 15A, b)

Trennung auf Al₂O₃- Ether /THF 9:1: Fraktionen 13 - 19: 2.35 g Substanz 15B.
Kristalle aus Diisopropylether/n-Hexan (1 mL, 1:1): 1.66 g.
Ausbeute: 6.6 %; C₂₂H₂₁FN₂O₂; MW = 364.42.
¹H-NMR (d6-DMSO) δ[ppm] =: 8.50 - 8.45 (AA', 2 H, pyridinyl); 7.45 - 7.37 (m, 2H , ar.); 7.30 - 7.15 (m, 4H, ar. + BB' pyridinyl); 3.80 (q, 2H, J= 7.0 Hz, OCH₂); 3.65 - 3.55 (m, 2H, CH₂ ); 2.75 - 2.65 (m, 2H CH₂) ; 1.80 - 1.60 (m, 4H, CH₂-CH₂) ; 0.757 (t, 3H, J = 7.0 Hz, CH₃).
¹³C-NMR (CDCl₃): δ[ppm] = 164.10, 162.09 (d, J = 243.4 Hz), 148.80, 143.29, 135.61, 132.840 (d, J = 8.4 Hz), 128.58, 127.816 (d, J = 3.3 Hz), 124.91, 117.61, 114.91 (d, J = 21.4 Hz), 111.33, 58.96, 44.35, 22.71, 22.34, 19.93, 13.52.

### Beispiel 16: [3-(4-Fluorphenyl)-2-(4-pyridyl)-5,6,7,8-tetrahydro-indolizin-1-yl]-methanol

Die Lösung von 3- (4-Fluorphenyl)-2-(4-pyridyl)-5,6,7,8-tetrahydro-indolizin-1-carbonsäure-ethylester (Beispiel 15 A, 2.55 g, 7 mMol) in Abs. THF (15 mL) wird bei RT unter Argon tropfenweise mit der Lösung von NaAlH₂(OC₂H₄OCH₃)₂ in Toluol (Vitride^{R} 70%, 2.9 mL, 10 mMol) versetzt und der Ansatz 24 h bei 40°C bis zum vollständigen Verschwinden des Eduktes (dc Al₂O₃/Ethyiacetat: Edukt rf = 0.8; Produkt rf = 0.5) gerührt. Hydridüberschuss wird durch Zugabe von Wasser (2 mL) zerstört, anschließend wird THF im Vakuum entfernt und der Rückstand in Ethylacetat aufgenommen. Die Ethylacetatphase wird mit Wasser bis zur neutralen Reaktion der Waschphasen gewaschen (60 mL). Die Ethylacetatphase wird über Na₂SO₄ sicc. getrocknet und eingeengt: es bleibt ein Rückstand von 2.15 g.
Ausbeute: 95 %; C₂₀H₁₉FN₂O; MW = 322.4.
IR (KBr) : 1/λ (cm⁻¹) = 3243, 2946, 1600, 1534, 1511, 1221, 994, 840, 579; -
¹H-NMR (CDCl₃) δ[ppm] = 8.40 - 8.37 / 7.15 - 7.12 (AA'BB'; 4H, - pyridyl); 7.17 - 7.12 (m; 2H, ar.); 7.07 - 6.98 (m; 2H, ar.); 4.55 (s; CH₂); 3.80 - 3.70 (m; CH₂) ; 3.00 - 2.90 (m; CH₂) ; 1.95 - 1.90 (m; 4H, CH₂) .

### Beispiel 17: [3-(4-Fluorphenyl)-1-methyl-2-(4-pyridyl)-5,6,7,8-tetrahydro-indolizin

Die Mischung von [3-(4-Fluorphenyl)-2-(4-pyridyl)-5,6,7,8-tetrahydro-indolizin-1-yl]-methanol (Beispiel 16, 3.52 g, 11 mMol) mit Iodwasserstoffsäure (57 %, 20 mL, 0.15 Mol) wird auf 100°C erhitzt und der Reaktionsverlauf dc (Al₂O₃, Ether) kontrolliert. Nach 45 min ist kein Edukt (rf = 0.2) mehr nachweisbar (Produkt: rf = 0.7).
Die abgekühlte Reaktionsmischung wird mit Ethylacetat (100 mL) versetzt, mit ges. Na₂CO₃-Lösung ausgerührt und mit Na₂S₂O₃-Lösung entfärbt und anschließend mit Wasser gewaschen. Die Ethylacetatphase wird über Na₂SO₄ sicc. getrocknet und eingeengt. Der Rückstand wird sc (Al₂O₃, Ether/THF 9:1) gereinigt:
Fraktion 4-11 liefert 1.6 g Produkt, das aus Diisopropylether kristallisiert: 1.02 g
Ausbeute: 30.4 %; C₂₀H₁₉FN₂; MW = 306.4.
¹H-NMR (CDCl₃) δ[ppm] = 8.40 - 8.36 / 7.00 - 6.97 (AA'BB', 4H, - pyridyl); 7.18-7.11 (m; 2H, ar.); 7.04 - 6.96 (m; 2H, ar.); 3.75 - 3.70 (m; CH₂); 2.83 - 2.78 (m; CH₂); 2.08 (s; 3H, CH₃) ; 1.95 - 1.85 (m; 4H, CH₂-CH₂); -
¹³C-NMR (CDCl₃): δ[ppm] = 164.5, 159.6, 149.1, 144.5, 132.6, 132.4, 128.6, 128.2, 128.1, 126.9, 124.6, 119.9, 115.7, 115.3, 11.9, 44.4, 23.8, 22.0, 20.9.

### Beispiel 18: 3-(4-Fluorphenyl)-2-(4-pyridyl)-5,6,7,8-tetrahydro-indolizin

3-(4-Fluorphenyl)-2-(4-pyridyl)-5,6,7,8-tetrahydro-indolizin-1-carbonsäure-ethylester (Beispiel 15A, 2.92 g, 8 mMol) und Iodwasserstoffsäure (57%, 16.0 mL, 27 g, 120 mMol) werden bei 60°C gemischt und auf 120°C temperiert. Das zunächst abgeschiedene Hydroiodid löst sich in der Hitze wieder (10 min). Der Reaktionsverlauf wird dc (Al₂O₃, Ether/THF 9:1) kontrolliert. Nach 30 min ist die Umwandlung vollständig (Edukt rf = 0.6; Produkt rf = 0.8). Die Mischung wird im Eisbad abgekühlt.

Der gebildete Niederschlag des kristallisierten Hydroiodids wird abgesaugt, durch Waschen mit Wasser (6-mal 5 mL) von anhaftender HI gereinigt und im Vakuum getrocknet (Exsiccator, P₂O₅). An Hydroiodid werden 2.68 g (79.5 %) isoliert.
Die Mutterlauge der Kristallisation wird mit NaOH neutralisiert und mit Ethylacetat extrahiert. Die Ethylacetatphase wird mit Na₂S₂O₃-Lösung entfärbt und anschließend mit Wasser gewaschen. Die Ethylacetatphase wird über Na₂SO₄ sicc. getrocknet und eingeengt: 0.44 g Produkt (Base).
Isolierung der Base:
5-(4-Fluorphenyl)-6-(4-pyridyl)-2,3-dihydro-1H-pyrrolizinhydroiodid (2.68 g, 9.2 mMol) wird in CH₂Cl₂ (150 mL) suspendiert und mit gesättigter NaHCO₃-Lösung (40 mL) intensiv gerührt. Die CH₂Cl₂-Phase wird anschließend abgetrennt, mit Na₂SO₄ sicc getrocknet und im Vakuum bis auf ein geringes Volumen (5 mL) eingeengt. Die gebildeten Kristalle werden mit Diisopropylether gewaschen und getrocknet: 1.66 g.
Ausbeute: 72.2 %, C₁₉H₁₇FN₂; MW = 292.36
¹H-NMR (CDCl₃) δ[ppm] = 8.32 - 8.29 / 7.01 - 6.98 ( AA'BB', 4H, -pyridyl); 7.30 - 7.21 (m; 2H, ar.); 7.16 - 7.06 (m; 2H, ar.); 6.21(s; 1H); 3,70 - 3.64(m, CH₂) ; 2.91 - 2.85 (m; CH₂) ; 1.98 - 1.80 (m; 4H, CH₂-CH₂); -
¹³C-NMR (CDCl₃): δ[ppm] = 165.0, 160.1, 149.3, 144.2, 132.8, 132.6, 130.4, 126.1, 128.5, 128.4, 121.6, 119,2, 116.1, 115.7, 104.2, 44.3, 23.6, 23.4, 20.9.

### Beispiel 19A: 2-(4-Fluorphenyl)-3-(4-pyridyl)-6,7-dihydro-5Hpyrrolizin-1-carbonsäureethylester

### a) 1-(Pyridin-4-carbonyl)-pyrrolidin-2-carbonsäure-benzylester

L-Prolin-benzylester-hydrochlorid (2.42 g, 0.01 Mol) gelöst in Pyridin (80 mL) wird bei 3°C (Eisbadkühlung) portionsweise mit Isonicotinsäurechlorid-Hydrochlorid (1.78 g, 0.01 Mol) versetzt, wobei sich die IT auf 10°C erhöht. Nach Entfernen des Eisbades wird 5 h gerührt , wobei sich der Ansatz grünlich verfärbt.

Überschüssiges Pyridin wird am Rotavapor entfernt und der erhaltene Rückstand zwischen Wasser (40 mL) und Ether verteilt. Nach Abtrennen der Etherphase wird die Wasserphase mit Ether (60 mL) extrahiert. Die Etherphasen werden gesammelt, mit Wasser (20 mL) gewaschen, getrocknet (Na₂SO₄ sicc.) und im Vakuum eingeengt. Der Rückstand wird im Hochvakuum getrocknet: 2.96 g.
Ausbeute: 85 %, C₁₈H₁₈N₂O₃, MW = 310.36;
¹H-NMR (CDCl₃) : δ[ppm] = 8.72 - 8.69 / 7.43 - 7.40 (AA'BB', 4H, -pyridyl); 7.37 (s; 5H); 5.260 / 5.188 ( AB, J_{AB} = 12.3 Hz, OCH₂Ph); 4.765 - 4.695 (m, CH_{X}, 1H); 3.610 - 3.395 (m, 2H, CH_{A}H_{B} ) : 2.140 - 1.875 (m, 4H, CH₂-CH_{A}H_{B}-CH_{X} ) .

### b) 1-(Pyridin-4-carbonyl)-pyrrolidin-2-carbonsäure

1-(Pyridin-4-carbonyl)-pyrrolidin-2-carbonsäure-benzylester (4.0 g, 11.5 mMol) wird bei RT in einem Gemisch aus THF und Ethanol abs. (1:1, 30 mL) gelöst, mit Palladium auf Aktivkohle (10%, 0.7 g) versetzt. Anschließend wird dreimal evakuiert und das Vakuum durch Wasserstoff aus einem Ballon ersetzt. Die Mischung wir 16 h bei atm und RT hydriert. Die Reaktionsmischung enthält daraufhin kein Edukt mehr (dc Al₂O₃ / n-Hexan Ethylacetat (1:1); Edukt: rf = 0.2-0.4 und Produkt: rf = 0.0).
Die Produktlösung wird vom Katalysator entfernt (G4), im Vakuum eingeengt und im Hochvakuum getrocknet, 2.95 g (>100%), Gehalt nach gc ca. 60 % ig.

### c) 2-Brom-3-(4-fluorphenyl)-propensäure-ethylester

Unter Lichtausschluss wird Brom-carbethoxymethylentriphenylphosporan (Beispiel 1A, b; 9.0 g, 21 mmol) in Toluol (60 mL) in Lösung gebracht und anschließend die Lösung von 4-Fluorbenzaldehyd (2.48 g, 20 mMol) in Toluol (9 mL) zugesetzt. Die Lösung wird im Dunkeln 16 h bei RT gerührt, im Vakuum eingeengt, der Rückstand mit Ether (40 mL) versetzt und die dabei abgeschiedenen Kristalle werden mit Ether (2x10 mL) gewaschen.

Die gesammelten Etherlösungen werden im Vakuum eingeengt und der verbleibende Rückstand (7.82 g) mit einem Ether/n-Hexan-Gemisch (1:1, 5 mL) versetzt. Dabei ungelöst zurückbleibender Niederschlag wird abgetrennt und mit Ether/n-Hexan-Gemisch (1:1) gewaschen. Beim Einengen verbleiben hier noch 6.06 g Rückstand die sc (SiO₂, Ether/n-Hexan 1:1) gereinigt werden:
Die gesuchte Substanz erscheint in den Fraktionen 1-2: gelbbraunes Öl, 4,72 g.
Ausbeute: 99.6 %; C₁₁H₁₀BrFO₂; MW = 273.10.; -
IR (NaCl): 1/λ (cm⁻¹) = 2983, 1724, 1601, 1508, 1261, 1235, 1195, 1161, 1040, 833; -
¹H-NMR (CDCl₃:) δ[ppm] = 8.18 (s; 1H) ; 7.91 - 7.84 (m, 2H, ar.); 7.16 - 7.07 (m; 2H, ar.) 4.36 (q; J=7.2Hz, CH₂) ; 1.38 (t; J=7.1Hz, CH₃); -
¹³C-NMR (CDCl₃:) δ[ppm] = 163.4 (d, J = 250 Hz), 163.2, 139.4, 138.7, 132.4 (d, J = 9 Hz), 129.9, 115.55 (d, 24 Hz), 112.9, 62.7, 15.1.

### d) 2-(4-Fluorphenyl)-3-(4-pyridyl)-6,7-dihydro-5H-pyrrolizin-1-carbonsäureethylester

Eine Gemisch aus 1-(Pyridin-4-carbonyl)-pyrrolidin-2-carbonsäure (Beispiel 19A, b; 60 %, 1.5 g, 4.1 mMol), 2-Brom-3-(4-fluorphenyl)-propensäure-ethylester (Beispiel 19A, c; 1.0 g, 4 mmol) und Acetanhydrid (10 mL) werden in einem auf 150°C temperierten Ölbad 21 h unter Rückfluss erhitzt. Nach Abkühlen und Einengen im Vakuum (Rotavapor) scheiden sich Nadeln ab. Die eingeengte Kristall-Suspension wird mit Ether/Ethylacetat (1:2) versetzt und die Kristalle abgesaugt. Die Mutterlauge wird vollständig eingeengt und der Rückstand sc (Al₂O₃- Ether /THF 9:1) gereinigt:
Fraktionen 3-5: Produkt 19 A, das aus Ether kristallisiert.
Die Kristalle werden abgesaugt und getrocknet: 0.12 g.
Ausbeute: 8.5 %, C₂₁H₁₉FN₂O₂; MW = 350.4.
¹H-NMR (CDCl₃) δ[ppm] = 8.45 - 8.42 / 6.97 - 6.94 (AA'BB'; 4H, - pyridyl); 7.23 - 7.15 / 7.02 - 6.93 (AA'BB'; 4H, ar.); 4.20 - 4.09 (q, CH₂O; + t, CH₂, 4H) ; 3.23 (t; J=7.5Hz, 2H); 2.58 (quin; J=7.2Hz, 2H, CH₂) ; 1.18 (t; J=7.1Hz, CH₃)

### Beispiel 19B: 1-(4-Fluorphenyl)-3-(4-pyridyl)-6,7-dihydro-5Hpyrrolizin-2-carbonsäureethylester

### SC Reinigung der Verbindung aus Beispiel 19A, d)

Trennung auf Al₂O₃- Ether /THF 9:1: Fraktionen 7 - 20, Kristalle aus Ether: 0.05 g.
Ausbeute: 3 %; C₂₁H₁₉FN₂O₂; MW = 350.4.
¹H-NMR (CDCl₃) δ[ppm] = 8.67 - 8.64 / 7.40-7.37 (AA'BB'; 4H, pyridyl); 7.39-7.32 / 6.98- 6.93 (m; 4H, ar.); 3.97 (t; 2H, J=7.1 Hz); 2.94 (t; 2H, J=7.2HZ); 2.62 - 2.48 (m; 2H); 0.975 (t; J=7.1Hz, CH₃) .

### Beispiel 20: [2-(4-Fluorphenyl)-3-(4-pyridyl)-6,7-dihydro-5Hpyrrolizin-1-yl]-methanol

Unter Argon wird 2-(4-Fluorphenyl)-3-(4-pyridyl)-6,7-dihydro-5H-pyrrolizin-1-carbonsäureethylester (Beispiel 19A , 0.15 g, 0.43 mMol) in abs. THF (4 mL) gelöst und bei 40°C unter Feuchtigkeitsausschluss Natrium-dihydrido-bis-(2-methoxyethoxy)-aluminat-Lösung (Vitride^{R}, 70 % in Toluol, 0.9 mL, 4.5 mMol) in 4 Portionen in jeweils 1 stündigen Abstand zugetropft. 1 h nach der letzten Zugabe ist dc (Al₂O₃ - Ethylacetat/n-Hexan 3:7) kein Edukt mehr nachzuweisen. Daraufhin lässt man abkühlen. Die Reaktionslösung wird zwischen Wasser (20 mL) und Ethylacetat (20 mL) verteilt, durch HCl (3 %) wird die Wasserphase auf pH 6-7 neutralisiert. Die Phasen werden getrennt, die Wasserphase wird 3-mal mit Ethylacetat (20 mL) ausgeschüttelt. Die vereinigte organischen Phase wird über Na₂SO₄ sicc getrocknet und im Vakuum eingeengt: 0.21 g.
Ausbeute: > 100 %; C₁₉H₁₇FN₂O; MW = 308.36.
¹H-NMR (CDCl₃) : δ[ppm] = 8.41 - 8.38 / 7.05 - 7.01 (AA'BB'; 4H, -pyridyl); 7.26 - 7.20 (m; 2H, ar.); 7.04 - 6.97 (m; 2H, ar.); 4.47 (s; CH₂O) ; 4.12 (t; J=7.0 Hz, CH₂N) ; 3.01 (t; J=7.3 Hz, CH₂); 2.57 (quin; J=7.5 Hz, 2H, CH₂) .

### Beispiel 21: 6-(4-Fluorphenyl)-7-methyl-5-(4-pyridyl)-2,3-dihydro-1H-pyrrolizin

### a) 2-Ethyl-1-pyrrolin

In einem 1 L 3-Halskolbenkolben mit Tropftrichter und Rückflusskühler wird Natriumhydrid (60 % in Paraffin, 36 g, 0.9 Mol) in abs. THF (180 mL) suspendiert und die Suspension für 10 min unter schwachem Rückflusskochen erwärmt. Die Mischung von Propionsäureethylester (33.7 g, 0.33 Mol) und 1-Vinyl-2-pyrrolidon (33.34 g, 0.3 Mol) in abs. THF (35 mL) werden zur siedenden Suspension getropft (10 min) und durch Erwärmen 3.5 h unter Rühren am Rückfluss gehalten.
Nach Abkühlung auf 10°C (Eisbad) wird mit ges. Ammoniumchlorid-Lösung (300 mL) der Natriumhydridüberschuss vernichtet (Cave! H₂) und neutralisiert und zum Austreiben des freiwerdenden Ammoniaks wird die inzwischen 30°C warme Mischung noch 10 min intensiv gerührt. Die abgeschiedene THF-Phase wird abgetrennt, über Na₂SO₄ sicc. getrocknet und eingeengt. Die sich auf der Ölphase abscheidende Paraffinschicht wird dekantiert. Die erhaltene rohe, ölige Produktfraktion (3-Propionyl-1vinyl-2-pyrrolidon, 51,2 g, ca 92 %) wird ohne weitere Reinigung zur Herstellung des 2-Ethyl-1-pyrrolin herangezogen:
Siedep.=140.20°C (760 Torr), Ausbeute: 102 %, C₉H₁₃NO₂, MW = 167.21.
IR (NaCl): 1/λ (cm⁻1) = 2955, 2925, 2854, 1698, 1633, 1456, 1427, 1387, 1327, 1273, 1114, 979; -
¹HNMR (CDCl₃) : d (ppm) = 7.08 - 6.95 (CH); 4.52 - 4.42 (CH₂); 3.74 - 3.67 (CH); 3.60 - 3.41 (CH₂); 3.13 - 2.96 (CH); 2.69 - 2.51 (CH₂); 2.23 - 2.09 (CH); 1.08 (t, J = 7 Hz, CH₃) .
¹³CNMR (CDCl₃) : d (ppm) = 205.3 (C=O), 168.4 (C=O), 129.1 (C-H), 95.6 (CH₂), 55.2 (C-H), 43.1 (CH₂), 35.9 (CH₂), 19.3 (CH₂), 7.3 (CH₃)

In einem 3-Halskolben-Kolben mit Tropftrichter und Wasserabscheider mit Rückflusskühler wird HCl (20 %, 300 mL) zum schwachen Sieden erhitzt. Aus dem Tropftrichter wird eine Lösung des rohen 3-Propionyl-1-vinyl-2-pyrrolidon (40.4g, 240 mMol) in THF (60 mL) zugetropft (10 min) und die Ansatzmischung bei 100°C (IT) gehalten. Das am Wasserabscheider gesammelte Acetaldehyd/THF-Gemisch (47 mL) wird verworfen. Die Mischung wird 6 h bei dieser Temperatur gehalten, abgekühlt und mit Ether (200 mL) extrahiert. In der Kälte (5-10 °C) wird aus der HCl-sauren Wasserphase das 2-Ethyl-1-pyrrolin durch Alkalisieren auf pH 9-10 abgeschieden. Das abgeschiedene Öl wird in Diethylether (150 mL) aufgenommen und die Wasserphase wird mit Diethylether (300 mL) extrahiert. Die Etherphasen werden vereint, getrocknet (K₂CO₃) und bei schwachem Vakuum (240 mmHg, 45°C) eingeengt. Man erhält 18.4 g 2-Ethyl-1-pyrrolin als gelbgefärbtes öl (ca 94 %).
Siedep.=109.5 (760 mmHg); Ausbeute: 79 %, C₆H₁₁N, MW = 97.16.
IR (NaCl) : 1/λ (cm⁻¹) 3378, 2969, 2937, 2870, 1644, 1462, 1454, 1431, 1371, 1300, 1144, 1093, 1019, 961; -
¹HNMR (CDCl₃): d (ppm) = 3.38 - 3.76 (m; CH₂); 2.52 - 2.34 (m; 2 CH₂); 1.89 (quin.; CH₂, J=7,8 Hz); 1.15 (t; CH₃, J=7,6Hz);
¹³CNMR (CDCl₃): d (ppm) = 179.8, 60.5, 36.9, 26.8, 22.4, 10.6.

### b) 6-(4-Fluorphenyl)-7-methyl-2,3-dihydro-1H-pyrrolizin

In einem 500 mL Kolben gibt man zum öligen Ethyl-1-pyrrolin (17.55 g, 180 mMol) in Anteilen 2-Brom-1-(4-fluorphenyl)-1-ethanon (19.53 g, 90 mMol) wobei man zwischen den Zugaben das exoterm reagierende Gemisch abkühlt. Die Mischung der Reaktionskomponenten wird im Ölbad (100 °C) unter Rühren erhitzt (30 min). Der Reaktionsverlauf wird dc überwacht.
Die abgekühlte Mischung wird mit CH₂Cl₂ (250 mL) versetzt und abgeschiedene Salze im Scheidetrichter mit zwei Portionen HCl (3 %, 40 mL) ausgewaschen. Die CH₂Cl₂-Phase wird mit Wasser (50 mL) gewaschen, getrocknet (Na₂SO₄ sicc.) und eingeengt.
Als Rückstand bleiben 14.04 g 6-(4-Fluorphenyl)-7-methyl-2,3-dihydro-1H-pyrrolizin als braunes zähes Öl (ca. 90 %).
Ausbeute: 72 %; C₁₄H₁₄FN, MW = 215.27.
¹HNMR (CDCl₃) : d (ppm) = 7.39 - 7.30 (m, 2H, F-ar.); 7.08 - 6.98 (m, 2H, F-ar.); 6.67 (s, 1H, 5-H); 3.954 (t; 2H, CH₂, J=7 Hz); 2.801 (t; 2H, CH₂, J=7 Hz) ; 2.487 (quin.; CH₂, J=7 Hz); 2.125 (s; 3H, CH₃);
¹³CNMR (CDCl₃) : d (ppm) = 161.04 (d, C-F, J=242 Hz), 135.58 (d, J=2.0 Hz), 133.51 (d, J=2 Hz), 128.87 (d, J = 7.5 Hz); 127.65; 115.025 (d, J = 20.9 Hz); 111.11 (Pyrrol-C-H); 106.71; 46.47; 27.44; 23.14; 10.86.

### c) 4-[2-(4-Fluorphenyl)-1-methyl-6,7-dihydro-5H-pyrrolizin-3-yl]-4H-pyridin-1-carbonsäureethylester

Die Lösung von 6-(4-Fluorphenyl)-7-methyl-2,3-dihydro-1Hpyrrolizin (1.72 g, 8.0 mMol) in CH₂Cl₂ (55 mL) wird zunächst bei 0°C (Eisbad) mit Pyridin (1.6 mL, 1.58 g, 20 mMol) und tropfenweise mit der Lösung von Chlorameisensäureethylester (.2.1 g, 19.5 mMol) in CH₂Cl₂ (25 mL) versetzt (10 min). Nach Temperaturanstieg auf 3-6°C wird das Kühlmittel entfernt und bei RT 1 h gerührt, danach 15 min auf Siedetemperatur (36 °C) erwärmt. Anschließend und nach einer weiteren Stunde Rückflusskochen werden jeweils 2 weitere Portionen Pyridin ( je 0.8 mL, 10 mMol) und Chlorameisensäure (je 1.05 g, 10 mMol) zugetropft und nach insgesamt 3 h die Reaktion abgebrochen. Die dunkel gefärbte Ansatzlösung wird auf Eiswasser (100 mL) gegossen, 15 min gerührt und anschließend die Phasen getrennt. Die Wasserphase wird mit CH₂Cl₂ (100 mL) extrahiert und die vereinigte organische Phase mit Wasser (50 mL) gewaschen, getrocknet (Na₂SO₄ sicc.) und eingeengt. Als Rückstand verbleiben 2.69 g dunkles Öl (91.9 % roh) das noch Reste nicht umgesetzten 6-(4-Fluorphenyl)-7-methyl-2,3-dihydro-1H-pyrrolizins enthält und ohne weitere Reinigung in der nächsten Stufe eingesetzt wurde.
Ausbeute: 91 %; C₂₂H₂₃FN₂O₂; MW = 366.44.

### d) 6-(4-Fluorphenyl)-7-methyl-5-(4-pyridyl)-2,3-dihydro-1Hpyrrolizin

In die bei RT erhaltene Lösung der Dihydropyridin-Verbindung aus Beispiel 21, c (1.83 g, 5 mMol) in tert-Butanol (30 mL) wird Kalium-tert.-Butylat (3.36 g, 30 mMol) gegeben und zunächst 1 h unter Luftzutritt bei RT, danach 1 h unter Reflux intensiv gerührt. Die Lösung wird eingeengt, der Rückstand mit Wasser (40 mL) aufgenommen und mit Ethylacetat (100 mL) extrahiert. Die Ethylacetat-Phase wird mit Wasser (20 mL) gewaschen, getrocknet (Na₂SO₄ sicc.) und eingeengt. Der Versuch diesen Rückstand (1.03 g) aus Ether (3 mL) zu kristallisieren gelang nicht.
Die Reinigung der Substanz erfolgte sc (Al₂O₃, Ether):
Der aus Fraktionen 33-42 erhaltene Rückstand kristallisiert aus Ether: 0.14 g.
Ausbeute: 10 %; C₁₉H₁₇FN₂; MW = 292.36
¹H-NMR (CDCl₃) δ[ppm] = 8.40 - 8.37 / 7.00-6.97 (AA'BB'; 4H, - pyridyl); 7.20 - 7.11 (m; 2H, ar.); 7.06 - 6.96 (m; 2H, ar.); 4.10 (t; J=7.0 Hz, CH₂); 2.885 (t; J=9.4 Hz, CH₂); 2.54 (quin; J=7.1 Hz, CH₂), 1.99 (s; CH₃) .

### Beispiel 22: 6-(4-Fluorphenyl)-5-(4-pyridyl)-2,3-dihydro-1Hpyrrolizin

### a) 1-Fluor-4-(2-nitrovinyl)-benzol

Die Mischung von 4-Fluorbenzaldehyd (12.4 g, 0.1 Mol), wasserfreiem Ammoniumacetat (7.8 g, 0.101 Mol) und Nitromethan (8.1 mL, 9.15 g, 0.15 Mol) in Eisessig (68 mL) werden 3.5 h zum Rückfluss (110 °C) erhitzt. Die abgekühlte Mischung wird mit Eiswasser (80 mL) versetzt, worauf braune Nadeln auskristallisieren. Nach Kaltstellen in einem Eisbad für 30 min werden die Kristalle abgesaugt, mit Wasser (60 mL) gewaschen und im Vakuum (Exsiccator) über P₂O₅ 16 h getrocknet: 13.2 g (81.8 %) .
Die Kristalle werden aus Essigsäure (50%, 130 mL) in der Wärme umkristallisiert: die in der Kälte kristallisierenden schwach hellgrünen Kristalle (2 Fraktionen) werden mit Wasser (60 mL) gewaschen und wie oben getrocknet: 9.76 g, und 1.09 g, Ausbeute: 67 % (10.8 g), C₈H₆FNO₂; MW = 167.14
IR (KBr): 1/λ (cm⁻¹) = 3111, 1637, 1595, 1501, 1341, 1230, 1164, 965, 827, 515; -
¹H-NMR (CDCl₃) : δ[ppm] = 8.02 - 7.96 (d, 1H, J=13.7Hz, - C_{b}H=CHₐNO₂); 7.60-7.50 (m, 3H, ar. + -C_{b}H=CHₐNO₂); 7.20 - 7.11 (t, 2H, J=8 . 5Hz, ar.).

### b) 6-(4-Fluorphenyl)-5-(4-pyridyl)-2,3-dihydro-1H-pyrrolizin

1-(Pyridin-4-carbonyl)-pyrrolidin-2-carbonsäure (Beispiel 19A, b; 60 %, 2.0 g, 5.5 mMol) bei 80°C gelöst in Acetanhydrid(16 mL) wird mit 1-Fluor-4-(2-nitrovinyl)-benzol (1.32 g, 7.9 mMol) versetzt und auf 130°C (IT) erhitzt. Bei 110°C beginnt die Freisetzung von NO₂ und CO₂, die nach 60 min beendet ist.
Nach insgesamt 2 h bei 110 -130°C lässt man den Ansatz abkühlen und für 16 h bei RT stehen.
Die Mischung wird zwischen Wasser (70 mL) und Ethylacetat (50 ml) verteilt, die organische Phase abgetrennt, die H₂O-Phase mit Ethylacetat extrahiert (100 mL) und die vereinigte Ethylacetat-Phase getrocknet (Na₂SO₄ sicc.) und eingeengt
Die Isolierung des Produktes erfolgt über SC (Al₂O₃, Ether):
Fraktionen 17 - 60, (rf = 0.5): Rückstand aus Diisopropylether kristallisiert, 0.32 g (Nadeln, 2. Kristallisationsfraktion aus der Mutterlauge 0.1 g).
Ausbeute: 16.6 % (0.32 g), C₁₈H₁₅FN₂; MW = 278.33
IR (KBr): 1/λ (cm⁻¹) = 3111, 1637, 1595, 1501, 1341, 1230, 1164, 965, 827, 515; -
¹H-NMR (CDCl₃) δ[ppm] = 8.49 - 8.45 / 7.13 - 7.10 (AA'BB'; 4H, - pyridyl); 7.25 - 7.17 (m; 2H, ar.); 6.99 - 6.90 (m; 2H, ar.); 6.03 (s; H, pyrrolizin-C5-H); 4.06 (t; J=7.0Hz, CH₂); 2.95 (t; J=7.3Hz, CH₂); 2.54 ( quin; J=7.2Hz, CH₂); -
¹³C-NMR (CDCl₃): δ[ppm] = 164.0, 159.2, 150.0 140.6, 139.6, 132.8, 132.7, 130.1, 130.0, 128.3, 122.8, 115.6, 115.2, 120.1, 46.9, 27.5, 24.5.

### Beispiel 23: 7-(4-Fluorphenyl)-3,3-dimethyl-8-pyridin-4-yl-3,4-dihydro-2H-pyrrolo[2,1-b] [1,3]-thiazin

### a) 4-(4-Fluorphenyl)-3-pyridin-4-yl-5H-furan-2-on

Zur Suspension von 4-Pyridylessigsäure-Hydrochlorid (25.9 g, 0.15 Mol) in wasserfreiem MeOH (150 mL) wird die Lösung von K-tert.-Butylat (33.7 g, 0.3 mMol) in MeOH (220 mL), die unter Eiskühlung bereitet wird, zugetropft. Nach 1 h Rühren bei RT wird der Methanol im Vakuum abdestilliert und der Rückstand der Kaliumsalze in DMF abs. (250 mL) aufgenommen. 2-Brom-1-(4-fluorphenyl)-ethanon (23.9 g, 0.11 Mol) wird in Portionen (5 g) eingerührt und die Mischung 2 h bei RT weitergerührt. Danach wird auf Wasser gegossen (1.5 L) und mit CHCl₃ (250 mL) 30 min ausgerührt und die CHCl₃-Phase im Scheidetrichter abgetrennt. Die abgetrennte Wasserphase wird mit CHCl₃ (300 mL) extrahiert, und die vereinigten CHCl₃-Extrakte mit Wasser (200 mL) gewaschen, über Na₂SO₄ sicc. getrocknet und eingeengt. Der dunkelgrüne halbkristalline Rückstand wird mit EtOH (50 mL) digeriert, die Kristalle abgesaugt, mit EtOH (20 mL) gewaschen und getrocknet: 18.8 g gelbgrüne Kristalle.
Schmp. 157.0 °C; Ausbeute: 50 %, C₁₅H₁₀FNO₂; MW = 255.25;
IR (KBr): 1/λ (cm⁻¹) = 1746, 1647, 1602, 1509, 1233, 1161, 1037, 827, 839; -
¹H-NMR (CDCl₃) : δ[ppm] = 8.68 - 8.64 (m, 2H, AA' pyridyl.); 7.38 - 7.26 (m, 4H, BB' pyridyl. + F-ar.); 7.14 - 7.05 (m, 2H, F-ar.); 5.19 (s, 2H, CH₂); -

### b) 4-(4-Fluorphenyl)-1-(3-hydroxy-2,2-dimethyl-propyl)-3-pyridin-4-yl-1,5-dihydro-pyrrol-2-on

Äquimolare Mengen an Neopentanolamin (3-Hydroxy-2,2-dimethylpropylamin, 36.3 g, 0.35 Mol) und Eisessig (21.0 g, 0.35 Mol) werden unter Wärmetönung homogen gemischt und die noch warme Mischung durch Einsenken in ein temperiertes Ölbad auf 105°C (IT) erhitzt. In diese Neopentanolammoniumacetat-Schmelze wird 4-(4-Fluorphenyl)-3-pyridin-4-yl-5H-furan-2-on (17.8 g, 0.07 Mol) in 4 Portionen eingerührt (15 min) und das Reaktionsgemisch noch 1.5 h erhitzt. Die abgekühlte Reaktionsmischung wird mit gesätt. NaHCO₃-Lösung und Ethylacetat (50 mL) versetzt und intensiv gerührt (30 min). Die abgeschiedenen Kristalle werden abgesaugt, mit Ethylacetat gewaschen und im Vakuum getrocknet.: 12.3 g.
Ausbeute: 52 %, C₂₀H₂₁FN₂O₂; MW = 340.40
IR (KBr) : 1/λ (cm⁻¹) = 3410, 1662, 1598, 1506, 1381, 1228, 1049, 831; -
¹H-NMR (CDCl₃) δ[ppm] = 8,63 - 8,59 (m, 2H, AA' pyridyl.); 7,351 - 7.225 (m, 4H, BB' pyridyl. + F-ar.); 7.085 - 6.995 (m, 2H, F-ar.); 4,44 (s, 2H, C-5-CH₂); 3.40 (s, 2H, CH₂OH); 3.27 (br, 2H, CH₂N); 1.88 (br, OH), 1.00 (s, 6H, CH₃) .

### c) 7-(4-Fluorphenyl)-3,3-dimethyl-8-pyridin-4-yl-3,4-dihydro-2H-pyrrolo[2,1-b] [1,3]-thiazin

4-(4-Fluorphenyl)-1-(3-hydroxy-2,2-dimethyl-propyl)-3-pyridin-4-yl-1,5-dihydro-pyrrol-2-on (5.1 g, 15 mMol) und Phosphorpentasulfid (P₂S₅, 2.5 g, 11.2 mMol werden innig verrieben und die Verreibung anschließend im Ölbad unter Argon 3 h auf 210°C erhitzt. Die erstarrte glasartige Masse wird in Ethylacetat (40 mL) und NaOH (10 %, 40 mL) aufgelöst. Die Ethylacetatphase wird abgetrennt, mit Wasser gewaschen, getrocknet (Na₂SO₄ sicc.) und eingeengt. Der Rückstand (3.8 g) wird sc (SiO₂/Ethylacetat) gereinigt:
Der aus Fraktion 2 (rf = 0.9) erhaltene Rückstand kristallisiert aus Diisopropylether: 0.6 g.
Ausbeute: 11.8 %, C₂₀H₁₉FN₂S; MW = 338.45
IR (KBr): 1/λ (cm⁻¹) = 3432, 1964, 1597, 1535, 1498, 1385, 1212, 1164, 991, 840, 816, 591, 517; -
¹H-NMR (CDCl₃) δ[ppm) = 8.465 - 8.430 (m, 2H, AA' pyridyl.); 7.262 - 7.05 (m, 4H, BB' pyridyl. + F-ar.); 7.00 - 6.935 (m, 2H, F-ar.); 6.71 (s, 1H, CH=); 3.70 (s, 2H, CH₂) ; 2,80 (s, 2H, CH₂); 1.22 (s, 6H, C(CH₃)₂); -
GC-MS (EI, 70 eV): m/z (rel Int. [%]) = 340 (9), 339 (28), 338 (100), 282 (8), 281 (30).

### Beispiel 24: 7-(4-Fluorphenyl)-3,3-dimethyl-8-pyridin-4-yl-3,4-dihydro-2H-pyrrolo[2,1-b][1,3]-oxazin

4-(4-Fluorphenyl)-1-(3-hydroxy-2,2-dimethyl-propyl)-3-pyridin-4-yl-1,5-dihydro-pyrrol-2-on (Beispiel 23, b; 2.55 g, 7.5 mMol) gelöst in CH₂Cl₂ (30 mL) wird mit Pyridin (2.82 g, 35.6 mMol) und anschließend tropfenweise mit Methansulfonsäurechloid (3.84 g, 33.8 mMol) versetzt und die Mischung 16 h bei RT gerührt. Wasser (20 mL) wird zugegeben und nach 15 min Rühren werden die Phasen im Scheidetrichter getrennt. Die CH₂Cl₂-Phase wird zunächst mit ges. NaHCO₃-Lsg. (10 mL) neutral gewaschen, über Na₂SO₄ sicc. getrocknet und eingeengt. Der erhaltene Rückstand (1.9 g, 51 %) des Dimethansulfonates (+ Monomethansulfonat) wird in methanolischer KOH (25 mL, 2N) aufgenommen und 16 h unter Argon zum Rückfluss erhitzt. Danach wird mit halbgesättigter Kochsalz-Lösung (150 mL) verdünnt und mit Ethylacetat extrahiert. Die Ethylacetat-Extrakte werden über Na₂SO₄ sicc. getrocknet und eingeengt. Der Rückstand (0.8 g) wird aus Diisopropylether kristallisiert, die Kristalle abgesaugt und im Vakuum getrocknet: 0.3 g Kristalle.
Schmp. 173.0 °C, Ausbeute: 12.4 %, C₂₀H₁₉FN₂O; MW = 322.39
IR (KBr) : 1/λ (cm⁻¹) = 2962, 2870, 1599, 1549, 1505, 1389, 1217, 1142, 1006, 992, 823, 835, 809, 580; -
¹H-NMR (CDCl₃) δ[ppm] = 8.371 - 8.340 (m, 2H, AA' pyridyl.); 7.262 - 7.10 (m, 4H, BB' pyridyl. + F-ar.); 7.05 - 6.90 (m, 2H, F-ar.); 6.19 (s, 1H, CH=); 3.95 (s, 2H, CH₂); 3.71 (s, 2H, CH₂) ; 1.185 (s, 6H, C(CH₃)₂); -
GC-MS (70 eV) m/e=322(100%); 293(>10%), 238.

## Patentansprüche

1. 4-Pyridyl- und 2,4-Pyrimidinyl-substituierte Pyrrolderivate und ihre Anwendung in der Pharmazie der Formel 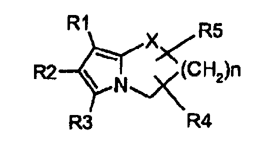 worin
einer der Reste R¹, R² und R³ für 4-Pyridyl , 2,4-Pyrimidyl oder 3-Amino-2,4-pyrimidin steht, das gegebenenfalls durch ein oder zwei C₁-C₄-Alkylgruppen oder Halogenatome substituiert ist,
der zweite der Reste R¹, R² und R³ für Phenyl oder Thienyl steht, das gegebenenfalls durch ein oder zwei C₁-C₄-Alkylgruppen oder Halogenatome substituiert ist, und
der dritte der Reste R¹, R² und R³ für H, CO₂H, CO₂C₁-C₆-Alkyl, CH₂OH oder C₁-C₆-Alkyl steht,
R⁴ und R⁵ unabhängig voneinander für H oder C₁-C₆-Alkyl stehen;
X für CH₂, S oder O steht und
n für 1 oder 2 steht,
und die optischen Isomere, physiologisch verträglichen Salze und physiologisch leicht hydrolysierbaren Ester davon.

2. Verbindungen der Formel I nach Anspruch 1, wobei der zweite der Reste R¹, R² und R³ für Fluor- oder Chlor-substituiertes Phenyl oder Thienyl steht.

3. Verbindungen der Formel I nach Anspruch 1, wobei der zweite der Reste R¹, R² und R³ für 4-Fluorphenyl oder 5-Chlorthien-2-yl steht.

4. Verbindungen der Formel I nach einem der vorhergehenden Ansprüche, wobei der dritte der Reste R¹, R² und R³ für CO2H steht.

5. Verbindungen der Formel I nach Anspruch 1, worin die Variablen die folgenden Bedeutungen haben: 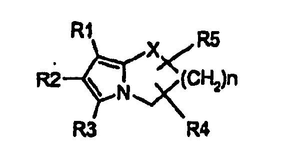
| X | n | R¹ | R² | R³ | R⁴ | R⁵ |
|---|---|---|---|---|---|---|
| CH₂ | 1 | CO₂Et | 4-Pyridyl | 4-F-Phenyl | H | H |
| CH₂ | 1 | 4-Pyridyl | CO₂Et | 4-F-Phenyl | H | H |
| CH₂ | 1 | CO₂H | 4-Pyridyl | 4-F-Phenyl | H | H |
| CH₂ | 1 | CH₂OH | 4-Pyridyl | 4-F-Phenyl | H | H |
| CH₂ | 1 | CH₃ | 4-Pyridyl | 4-F-Phenyl | H | H |
| CH₂ | 1 | H | 4-Pyridyl | 4-F-Phenyl | H | H |
| CH₂ | 1 | CO₂Et | 4-Pyridyl | 2-thienyl- | H | H |
| CH₂ | 1 | 4-Pyridyl | CO₂Et | 2-thienyl- | H | H |
| CH₂ | 1 | CO₂H | 4-Pyridyl | 2-thienyl- | H | H |
| CH₂ | 1 | H | 4-Pyridyl | 2-thienyl- | H | H |
| CH₂ | 1 | CO₂Eₜ | 4-Pyridyl | 5-Cl-2-thienyl- | H | H |
| CH₂ | 1 | 4-Pyridyl | CO₂Et | 5-Cl-2-thienyl- | H | H |
| S | 1 | CO₂Et | 4-Pyridyl | 4-F-Phenyl | H | H |
| S | 1 | 4-Pyridyl | CO₂Et | 4-F-Phenyl | H | H |
| S | 1 | CO₂H | 4-Pyridyl | 4-F-Phenyl | H | H |
| S | 1 | CH₂OH | 4-Pyridyl | 4-F-Phenyl | H | H |
| S | 1 | CH3 | 4-Pyridyl | 4-F-Phenyl | H | H |
| S | 1 | H | 4-Pyridyl | 4-F-Phenyl | H | H |
| CH₂ | 2 | CO₂Et | 4-Pyridyl | 4-F-Phenyl | H | H |
| CH₂ | 2 | 4-Pyridyl | CO₂Et | 4-F-Phenyl | H | H |
| CH₂ | 2 | CH₂OH | 4-Pyridyl | 4-F-Phenyl | H | H |
| CH₂ | 2 | CH₃ | 4-Pyridyl | 4-F-Phenyl | H | H |
| CH₂ | 2 | H | 4-Pyridyl | 4-F-Phenyl | H | H |
| CH₂ | 1 | CO₂Et | 4-F-Phenyl | 4-Pyridyl | H | H |
| CH₂ | 1 | 4-F-Phenyl | CO₂Et | 4-Pyridyl | H | H |
| CH₂ | 1 | CH₂OH | 4F-Phenyl | 4-Pyridyl | H | H |
| CH₂ | 1 | CH₃ | 4-F-Phenyl | 4-Pyridyl | H | H |
| CH₂ | 1 | H | 4.F-Phenyl | 4-Pyridyl | H | H |
| S | 2 | 4-Pyridyl | 4-F-Phenyl | H | CH₃ | CH₃ |
| O | 2 | 4-Pyridyl | 4-F-Phenyl | H | CH₃ | CH₃ |
| CH₂ | 1 | Phenyl | 4-F-Phenyl | 4.Pyridyl | H | H |
| CH₂ | 1 | Phenyl | 2,4-Pyrimidyl | CH₃ | H | H |
| CH₂ | 1 | 4-F-Phenyl | 2,4-Pyrimidyl | CH₃ | H | H |
| CH₂ | 1 | CH₃ | 4-F-Phenyl | 2,4-Pyrimidyl | H | H |
| CH₂ | 1 | Phenyl | 3-Amino-2,4-Pyrimidyl | CH₃ | H | H |
| CH₂ | 1 | 4-F-Phenyl | 3-Amino-2,4-Pyrimidyl | CH₃ | H | H |
| CH₂ | 1 | CH₃ | 4-F-Phenyl | 3-Amino-2,4-Pyrimidyl | H | H |

6. Pharmazeutisches Mittel enthaltend wenigstens eine Verbindung nach einem der Ansprüche 1 bis 5, gegebenenfalls zusammen mit pharmazeutisch akzeptablen Träger- und/oder Zusatzstoffen.

7. Verwendung wenigstens einer Verbindung nach einem der Ansprüche 1 bis 5 zur Herstellung eines pharmazeutischen Mittels zur Behandlung von Erkrankungen, die mit einer Störung des Immunsystems im Zusammenhang stehen.

8. Verwendung nach Anspruch 7 zur Behandlung von Autoimmunerkrankungen, Krebs, Multipler Sklerose, Arthritis, Inflammatory Bowel Disease, Septischem Schock, Adult Respiratory Distress Syndrome, und bei Transplantationen.

## Claims

1. 4-Pyridyl- or 2,4-pyrimidinyl-substituted pyrrole derivatives and their use in pharmacy, of the formula 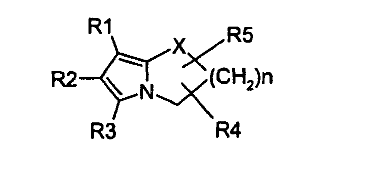 in which
one of the radicals R¹, R² and R³ is 4-pyridyl, 2,4-pyrimidyl or 3-amino-2,4-pyrimidyl, which is optionally substituted by one or two C₁-C₄-alkyl groups or halogen atoms,
the second of the radicals R¹, R² and R³ is phenyl or thienyl, which is optionally substituted by one or two C₁-C₄-alkyl groups or halogen atoms, and
the third of the radicals R¹, R² and R³ is H, CO₂H, CO₂C₁-C₆-alkyl, CH₂OH or C₁-C₆-alkyl,
R⁴ and R⁵ independently of one another are H or C₁-C₆-alkyl;
X is CH₂, S or O and
n is 1 or 2,
and the optical isomers, physiologically tolerable salts and physiologically easily hydrolyzable esters thereof.

2. The compounds of the formula I as claimed in claim 1, where the second of the radicals R¹, R² and R³ is fluorine- or chlorine-substituted phenyl or thienyl.

3. The compounds of the formula I as claimed in claim 1, where the second of the radicals R¹, R² and R³ is 4-fluorophenyl or 5-chlorothien-2-yl.

4. The compounds of the formula I as claimed in one of the preceding claims, where the third of the radicals R¹, R² and R³ is CO₂H.

5. The compounds of the formula I as claimed in claim 1, in which the variables have the following meanings: 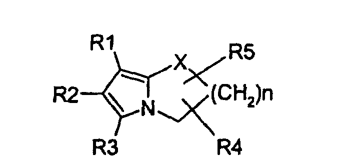
| X | n | R¹ | R² | R³ | R⁴ | R⁵ |
|---|---|---|---|---|---|---|
| CH₂ | 1 | CO₂Et | 4-pyridyl | 4-F-phenyl | H | H |
| CH₂ | 1 | 4-pyridyl | CO₂Et | 4-F-pheny | H | H |
| CH₂ | 1 | CO₂H | 4-pyridyl | 4-F-phenyl | H | H |
| CH₂ | 1 | CH₂OH | 4-pyridyl | 4-F-phenyl | H | H |
| CH₂ | 1 | CH₃ | 4-pyridyl | 4-F-phenyl | H | H |
| CH₂ | 1 | H | 4-pyridyl | 4-F-phenyl | H | H |
| CH₂ | 1 | CO₂Eₜ | 4-pyridyl | 2-thienyl- | H | H |
| CH₂ | 1 | 4-pyridyl | CO₂Et | 2-thienyl- | H | H |
| CH₂ | 1 | CO₂H | 4-pyridyl | 2-thienyl- | H | H |
| CH₂ | 1 | H | 4-pyridyl | 2-thienyl- | H | H |
| CH₂ | 1 | CO₂Et | 4-pyridyl | 5-Cl-2-thienyl- | H | H |
| CH₂ | 1 | 4-pyridyl | CO₂Eₜ | 5-Cl-2-thienyl- | H | H |
| S | 1 | CO₂Et | 4-pyridyl | 4-F-phenyl | H | H |
| S | 1 | 4-pyridyl | CO₂Eₜ | 4-F-pheny | H | H |
| S | 1 | CO₂H | 4-pyridyl | 4-F-phenyl | H | H |
| S | 1 | CH₂OH | 4-pyridyl | 4-F-phenyl | H | H |
| S | 1 | CH3 | 4-pyridyl | 4-F-phenyl | H | H |
| S | 1 | H | 4-pyridyl | 4-F-phenyl | H | H |
| CH₂ | 2 | CO₂Et | 4-pyridyl | 4-F-phenyl | H | H |
| CH₂ | 2 | 4-pyridyl | CO₂Et | 4-F-phenyl | H | H |
| CH₂ | 2 | CH₂OH | 4-pyridyl | 4-F-phenyl | H | H |
| CH₂ | 2 | CH₃ | 4-pyridyl | 4-F-phenyl | H | H |
| CH₂ | 2 | H | 4-pyridyl | 4-F-phenyl | H | H |
| CH₂ | 1 | CO₂Et | 4-F-phenyl | 4-pyridyl | H | H |
| CH₂ | 1 | 4-F-phenyl | CO₂Et | 4-pyridyl | H | H |
| CH₂ | 1 | CH₂OH | 4-F-phenyl | 4-pyridyl | H | H |
| CH₂ | 1 | CH₃ | 4-F-phenyl | 4-pyridyl | H | H |
| CH₂ | 1 | H | 4-F-phenyl | 4-pyridyl | H | H |
| S | 2 | 4-pyridyl | 4-F-phenyl | H | CH₃ | CH₃ |
| O | 2 | 4-pyridyl | 4-F-phenyl | H | CH₃ | CH₃ |
| CH₂ | 1 | phenyl | 4-F-phenyl | 4-pyridyl | H | H |
| CH₂ | 1 | phenyl | 2,4-pyrimidyl | CH₃ | H | H |
| CH₂ | 1 | 4-F-phenyl | 2,4-pyrimidyl | CH₃ | H | H |
| CH₂ | 1 | CH₃ | 4-F-phenyl | 2,4-pyrimidyl | H | H |
| CH₂ | 1 | phenyl | 3-amino-2,4-pyrimidyl | CH₃ | H | H |
| CH₂ | 1 | 4-F-phenyl | 3-amino-2,4-pyrimidyl | CH₃ | H | H |
| CH₂ | 1 | CH₃ | 4-F-phenyl | 3-amino-2,4-pyrimidyl | H | H |

6. A pharmaceutical composition comprising at least one compound as claimed in one of claims 1 to 5, if . appropriate together with pharmaceutically acceptable vehicles and/or additives.

7. The use of at least one compound as claimed in one of claims 1 to 5 for the preparation of a pharmaceutical composition for treating disorders which are connected with a disturbance of the immune system.

8. The use of claim 7 for the treatment of autoimmune diseases, cancer, multiple sclerosis, arthritis, inflammatory bowel disease, septic shock, adult respiratory distress syndrome, and in transplantations.

## Revendications

1. 4-Pyridyl- et 2,4-pyrimidinylpyrroles de formule (I) 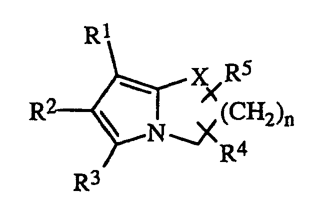 dans laquelle
un des symboles R¹, R² et R³ représente un résidu 4-pyridyle, 2,4-pyrimidyle ou 3-amino-2,4-pyrimidinyle, portant éventuellement un ou deux substituants alkyle en C₁₋₄ ou halogéno, le deuxième des symboles R¹_{,} R² et R³ représente un groupe phényle ou thiényle, portant éventuellement un ou deux substituants alkyle en C₁₋₄ ou halogéno, et le troisième des symboles R¹, R² et R³ représente un atome d'hydrogène ou un groupe -CO₂H, -CO₂(alkyle en C₁₋₆), -CH₂OH ou alkyle en C₁₋₆,
R⁴ et R⁵ représentent indépendamment l'un de l'autre un atome d'hydrogène ou un groupe alkyle en C₁₋₆,
X représente un groupe CH₂, S ou O et
n vaut 1 ou 2,
les isomères optiques, les sels physiologiquement acceptables et les esters facilement hydrolysables en milieu physiologique,
ainsi que leur utilisation dans le domaine pharmaceutique.

2. Composés de formule I selon la revendication 1, dans lesquels le deuxième des symboles R¹, R² et R³ représente résidu phényle ou thiényle fluoré ou chloré.

3. Composés de formule I selon la revendication 1, dans lesquels le deuxième des résidus R¹, R² et R³ représente un groupe 4-fluorophényle ou 5-chloro-thién-2-yle.

4. Composés de formule (I) selon l'une des revendications précédentes, dans lequel le troisième des symboles R¹_{,} R² et R³ représente un groupe -CO₂H.

5. Composés de formule (I) selon la revendication 1, dans lequel les symboles ont les significations suivantes : 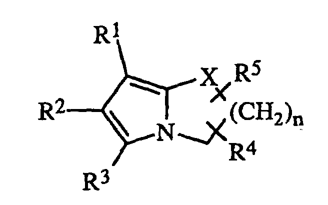
| X | n | R¹ | R² | R³ | R⁴ | R⁵ |
|---|---|---|---|---|---|---|
| CH₂ | 1 | CO₂Et | 4-pyridyle | 4-F-phényle | H | H |
| CH₂ | 1 | 4-pyridyle | CO₂Et | 4-F-phényle | H | H |
| CH₂ | 1 | CO₂H | 4-pyridyle | 4-F-phényle | H | H |
| CH₂ | 1 | CH₂OH | 4-pyridyle | 4-F-phényle | H | H |
| CH₂ | 1 | CH₃ | 4-pyridyle | 4-F-phényle | H | H |
| CH₂ | 1 | H | 4-pyridyle | 4-F-phényle | H | H |
| CH₂ | 1 | CO₂Eₜ | 4-pyridyle | 2-thiényle | H | H |
| CH₂ | 1 | 4-pyridyle | CO₂Et | 2-thiényle | H | H |
| CH₂ | 1 | CO₂H | 4-pyridyle | 2-thiényle | H | H |
| CH₂ | 1 | H | 4-pyridyle | 2-thiényle | H | H |
| CH₂ | 1 | CO₂Et | 4-pyridyle | 5-Cl-2-thiényle | H | H |
| CH₂ | 1 | 4-pyridyle | CO₂Et | 5-Cl-2-thiényle | H | H |
| S | 1 | CO₂Et | 4-pyridyle | 4-F-phényle | H | H |
| S | 1 | 4-pyridyle | CO₂Et | 4-F-phényle | H | H |
| S | 1 | CO₂H | 4-pyridyle | 4-F-phényle | H | H |
| S | 1 | CH₂OH | 4-pyridyle | 4-F-phényle | H | H |
| S | 1 | CH₃ | 4-pyridyle | 4-F-phényle | H | H |
| S | 1 | H | 4-pyridyle | 4-F-phényle | H | H |
| CH₂ | 2 | CO₂Et | 4-pyridyle | 4-F-phényle | H | H |
| CH₂ | 2 | 4-pyridyle | CO₂Et | 4-F-phényle | H | H |
| CH₂ | 2 | CH₂OH | 4-pyridyle | 4-F-phényle | H | H |
| CH₂ | 2 | CH₃ | 4-pyridyle | 4-F-phényle | H | H |
| CH₂ | 2 | H | 4-pyridyle | 4-F-phényle | H | H |
| CH₂ | 1 | CO₂Et | 4-F-phényle | 4-pyridyle | H | H |
| CH₂ | 1 | 4-F-phényle | CO₂Et | 4-pyridyle | H | H |
| CH₂ | 1 | CH₂OH | 4-F-phényle | 4-pyridyle | H | H |
| CH₂ | 1 | CH₃ | 4-F-phényle | 4-pyridyle | H | H |
| CH₂ | 1 | H | 4-F-phényle | 4-pyridyle | H | H |
| S | 2 | 4-pyridyle | 4-F-phényle | H | CH₃ | CH₃ |
| O | 2 | 4-pyridyle | 4-F-phényle | H | CH₃ | CH₃ |
| CH₂ | 1 | phényle | 4-F-phényle | 4-pyridyle | H | H |
| CH₂ | 1 | phényle | 2,4-pyrimidyle | CH₃ | H | H |
| CH₂ | 1 | 4-F-phényle | 2,4-pyrimidyle | CH₃ | H | H |
| CH₂ | 1 | CH₃ | 4-F-phényle | 2,4-pyrimidyle | H | H |
| CH₂ | 1 | phényle | 3-amino-2,4-pyrimidyle | CH₃ | H | H |
| CH₂ | 1 | 4-F-phényle | 3-amino-2,4-pyrimidyle | CH₃ | H | H |
| CH₂ | 1 | CH₃ | 4-F-phényl | 3-amino-2,4-pyrimidyle | H | H |

6. Composition pharmaceutique contenant au moins un composé selon l'une des revendications 1 à 5, éventuellement en combinaison avec un ou plusieurs véhicules et/ou additifs pharmaceutiques.

7. Utilisation d'au moins un des composés selon l'une des revendications 1 à 5 pour la préparation d'une composition pharmaceutique destinée au traitement de maladies liées à une perturbation du système immunitaire.

8. Utilisation selon la revendication 7, pour le traitement de maladies auto-immunes, du cancer, de la sclérose en plaques, de l'arthrite, des maladies inflammatoires de l'intestin (maladie de Crohn), du choc sceptique, du syndrome de détresse respiratoire chez l'adulte, et pour des greffes.
